# FASCICULE DE BREVET EUROPEEN

(11) **EP 2 307 369 B1**
(45) Date de publication et mention de la délivrance du brevet: **11.07.2018**
(21) Numéro de dépôt: 09740395.0
(22) Date de dépôt: 28.07.2009
(51) Int. Cl.: C07D 213/24, C07D 233/32, C07D 333/24, C07D 495/04, G01N 33/58

(54) **RÉACTIFS DE MARQUAGE AYANT UN NOYAU PYRIDINE PORTANT UNE FONCTION DIAZOMÉTHYLE, PROCÉDÉS DE SYNTHÈSE DE TELS RÉACTIFS ET PROCÉDÉS DE DÉTECTION DE MOLECULES BIOLOGIQUES**
MARKERREAGENZIEN MIT EINEM PYRIDINRING MIT EINEM SUBSTITUENTEN MIT DIAZOMETHYLFUNKTION, VERFAHREN ZUR SYNTHESE DIESER REAGENZIEN UND VERFAHREN ZUM NACHWEIS BIOLOGISCHER MOLEKÜLE
MARKER REAGENTS WITH A PYRIDINE RING WITH A DIAZOMETHYL FUNCTION SUBSTITUENT METHODS FOR SYNTHESIS OF SAID REAGENTS AND METHODS FOR DETECTING BIOLOGICAL MOLECULES

(30) Priorité: 29.07.2008 FR 0855190
(43) Date de publication de la demande: 13.04.2011
(73) Titulaire: Biomérieux, 69280 Marcy L'etoile (FR); Université de Strasbourg, 67081 Strasbourg Cedex (FR); CNRS, 75794 Paris Cedex 16 (FR)
(72) Inventeur: LAURENT, Alain, F-38100 Grenoble (FR); LAAYOUN, Ali, F-38260 La Frette (FR); KOTERA, Mitsuhara, F-67000 Strasbourg (FR)
(86) Numéro de dépôt international: PCT/FR2009/051511
(87) Numéro de publication internationale: WO 2010/012949

(56) Documents cités:
- EP-A1- 0 065 039
- WO-A-02/090319
- WO-A-02/090584
- WO-A-2005/092910
- WO-A-2009/001017
- SHIGA M ET AL: "SYNTHESIS OF A NOVEL BIOTIN DERIVATIVE THAT BEARS A DIAZO GROUP AS THE REACTIVE SITE" ANALYTICAL SCIENCES, JAPAN SOCIETY FOR ANALYTICAL CHEMISTRY, TOKYO, JP, vol. 9, no. 4, 1 août 1993 (1993-08-01), pages 553-556, XP008005959 ISSN: 0910-6340
- LAAYOUN A ET AL: "Aryldiazomethanes for Universal Labeling of Nucleic Acids and Analysis on DNA Chips" BIOCONJUGATE CHEMISTRY, ACS, WASHINGTON, DC, US, vol. 14, 1 janvier 2003 (2003-01-01), pages 1298-1306, XP002308387 ISSN: 1043-1802
- SHIGA M ET AL: "FLUORESCENCE DETECTION OF DNA USING A NOVEL PEROXIDASE SUBSTRATE, 4-(4-HYDROXYPHENYLCARBAMOYL)BUTANOIC ACID" ANALYTICAL SCIENCES, JAPAN SOCIETY FOR ANALYTICAL CHEMISTRY, TOKYO, JP, vol. 11, no. 4, 1 août 1995 (1995-08-01), pages 591-595, XP008005960 ISSN: 0910-6340
- TRYFIATES GEORGE P ET AL: "Synthesis of adenosine-N6-methyl, propylthioether-N-pyridoxamine: an analog of a novel vitamin B6 tumor product", IN VIVO - INTERNATIONAL JOURNAL OF IN VIVO RESEARCH,, vol. 3, no. 3, 1 January 1989 (1989-01-01) , pages 177-182, XP009164253, ISSN: 0258-851x
- Huw M. L. Davies ET AL: "Catalytic Asymmetric Cyclopropanation of Heteroaryldiazoacetates", The Journal of Organic Chemistry, vol. 66, no. 20, 1 October 2001 (2001-10-01), pages 6595-6603, XP055117701, ISSN: 0022-3263, DOI: 10.1021/jo015617t
- Bernd Eistert ET AL: "Über das Diazoketon "Azi-[alpha]-pyridil"", Chemische Berichte, vol. 91, no. 7, 1 July 1958 (1958-07-01), pages 1411-1415, XP055117698, ISSN: 0009-2940, DOI: 10.1002/cber.19580910710
- MA M ET AL: "An efficient synthesis of aryl alpha-keto esters", TETRAHEDRON LETTERS, PERGAMON, GB, vol. 46, no. 22, 30 May 2005 (2005-05-30), pages 3927-3929, XP027864049, ISSN: 0040-4039 [retrieved on 2005-05-30]
- FOULON C F ET AL: "SYNTHESIS AND PRELIMINARY BIOLOGICAL EVALUATION OF (3-IODOBENZOYL)NORBIOTINAMIDE AND ((5-IODO-3-PYRIDINYL)CARBONYL)NORBIOTINAMI DE: TWO RADIOIODINATED BIOTIN CONJUGATES WITH IMPROVED STABILITY", BIOCONJUGATE CHEMISTRY, ACS, WASHINGTON, DC, US, vol. 8, no. 2, 1 March 1997 (1997-03-01), pages 179-186, XP000682495, ISSN: 1043-1802, DOI: 10.1021/BC970006M

## Description

La présente invention concerne de nouveaux réactifs de marquage de molécules biologiques, un procédé de synthèse desdits marqueurs ainsi que des applications pour le marquage de molécules biologiques en particulier dans le domaine du diagnostic moléculaire utilisant la détection et l'analyse des acides nucléiques.

L'état de la technique montre que de nombreuses méthodes existent pour marquer des nucléotides, des oligonucléotides ou des acides nucléiques naturels ou amplifiés.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer un marquage d'un nucléotide ou d'un analogue de nucléotide ou d'un acide nucléique, est enclin à effectuer cette fixation sur la base ou sur le sucre qui lui offrent plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16094, DE-A-3.910.151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem. Soc., 82, 1778-1785, 1960). De même dans la revue de O' Donnel et Mc Laughlin (« Reporter groups for the analysis of nucleic acid structure », p 216-243, dans « Bioorganic Chemistry : Nucleic Acids », Ed. Hecht S.M., Oxford University Press, 1996) portant sur les méthodes d'introduction de sondes dans les fragments d'oligonucléotides, l'alkylation efficace du phosphodiester internucléotidique est considérée comme étant impossible.

La demande de brevet WO-A-99/65926 décrit un procédé de marquage d'un acide ribonucléique (ARN) de synthèse ou naturel qui consiste à fragmenter l'ARN et à marquer au niveau du phosphate terminal. Ce document décrit un certain nombre de fonctions pouvant être utilisées pour le marquage en liaison avec la fragmentation comme les fonctions hydroxyle, amine, hydrazine, alcoxyamine, halogénure d'alkyle, halogénure d'alkyle de type benzylique et en particulier le dérivé 5-(bromométhyl)fluorescéine. Ces fonctions permettent de marquer les acides nucléiques, mais il faut associer une étape de fragmentation pour avoir un marquage efficace car ce marquage se produit sur le phosphate libéré lors de la fragmentation. De plus, il faut ajouter un excès important de réactif de marquage par rapport à l'ARN pour obtenir un marquage efficace ce qui induit des problèmes de bruit de fond, lors de la détection, générés par le marqueur en excès. Enfin, cette méthode ne fonctionne pas efficacement sur de l'ADN double brin.

Il existe donc un besoin pour de nouveaux réactifs qui soient efficaces du point de vue du rendement de marquage, qui soient spécifiques au niveau de la position de marquage et en particulier qui n'affectent pas les propriétés d'hybridation des bases impliquées dans la formation de la double hélice, par l'intermédiaire des liaisons hydrogènes, qui soient utilisables à la fois pour l'ADN et l'ARN, et enfin qui permettent de marquer indifféremment des nucléotides, des oligonucléotides, des acides nucléiques naturels ou préparés par transcription, par transcription inversée ou par amplification enzymatique.

La Demanderesse a déjà proposé de tels nouveaux marqueurs qui répondent aux conditions précitées et qui utilisent la fonction diazométhyle comme fonction réactive pour le marquage. C'est par exemple le cas dans les demandes de brevet WO-A-02/090319, WO-A-02/090584 et WO-A-2005/092910.

Ainsi la fonction diazométhyle (de formule -C(N₂)-) a déjà été utilisée pour l'alkylation des groupements phosphates, mais un certain nombre de problèmes se pose. D'une part, les réactifs incorporant au moins une fonction diazo en général sont instables par eux-mêmes, ce qui pose des problèmes pour l'utilisation de ces réactifs dans un kit de marquage, ce qui est rédhibitoire si le produit marqué a pour fonction de mettre en évidence la présence d'une molécule cible biologique dans un échantillon quelconque.

Enfin les réactifs portant la fonction diazométhyle, portée par un noyau aromatique du type phényle, pour assurer sa stabilité, sont associés à certains marqueurs, tels que la biotine. La présence du noyau aromatique et la nature du marqueur rendent ces réactifs peu solubles dans l'eau, ce qui conduit à utiliser des solvants organiques qui sont miscibles à l'eau pour le couplage avec des molécules biologiques, qui ne sont solubles que dans l'eau ou dans des tampons aqueux. Mais ces solvants, s'ils sont utilisés en concentration trop importante dans la réaction de marquage, risquent de provoquer la précipitation des biomolécules. De plus, pour l'automatisation des protocoles de marquage dans des dispositifs intégrés, il est indispensable de lyophiliser les réactifs de marquage dans les tampons ne contenant pas de solvants organiques. Ces derniers ne sont pas compatibles avec la lyophilisation. Il convient donc de pouvoir disposer de réactifs de marquage suffisamment solubles et stables dans les milieux aqueux.

Les réactifs de marquage préconisés par les documents WO-A-02/090319, WO-A-02/090584 (molécules de première génération) et WO-A-2005/092910 (molécules de deuxième génération) ci-dessus mentionnés résolvent aussi ces problèmes techniques.

Ces molécules ont donc les inconvénients suivants :
- instables chimiquement, car elles doivent être à sec ou en solution à 4°C,
- synthèse relativement complexe, et
- peu solubles dans une solution aqueuse.

Malgré ces inconvénients, ces molécules sont particulièrement intéressantes et attractives pour l'homme du métier. Il est toutefois possible d'améliorer leurs caractéristiques chimiques ou physiques, c'est ce qui a prévalu lors de l'élaboration des molécules de troisième génération, exposées dans le document WO-A-2009/001017, dans laquelle les molécules sont à la fois plus stables et plus faciles à synthétiser.

Ce début de solution a permis de faire face au problème de la stabilité des molécules de première et de deuxième générations, toutefois leur solubilité est un peu moins bonne que les molécules de deuxième génération (0,3 mM dans 100% d'eau). Quoiqu'il en soit la Demanderesse continue aujourd'hui dans cette voie avec la description d'un nouveau type de molécules bien plus solubles que les molécules de troisième génération (solubilité comprise entre 2 et 28 mM dans 100% d'eau) et d'une stabilité égale ou supérieure aux molécules déjà décrite dans nos précédentes inventions.

Ces quatre documents, ci-dessus exposés, présentent les trois précédentes générations de molécules, qui ont toutes pour base la présence d'une fonction diazométhyle à proximité d'un cycle. Le lecteur est invité à se référer à ces quatre documents pour toute explication complémentaire qui viendrait, par omission involontaire, à manquer dans le texte exposant la présente invention.

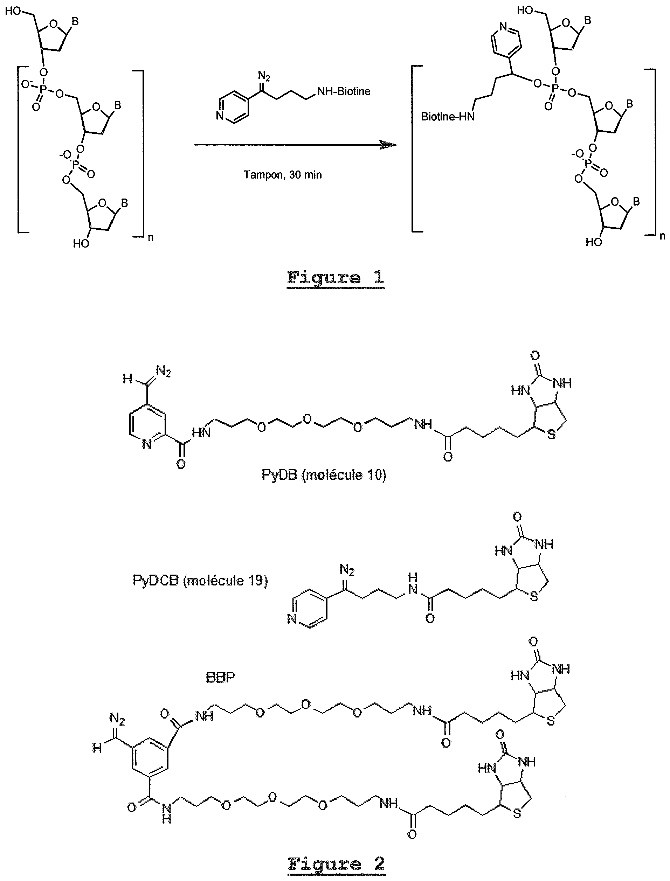

Pour cela, la nouvelle invention propose d'introduire un noyau pyridine en *alpha* de la fonction diazo méthyle, le groupement de reconnaissance étant soit en *alpha'*, soit sur le cycle pyridinyle (Voir Figure 2, molécule 10 ou molécule 19). Les molécules de quatrième génération proposent le meilleur profil combinant stabilité et solubilité.

Ceci apporte à la molécule :
1) une stabilité chimique supérieure ou égale aux Nitro DCB, sous forme sèche à 4°C ou en solution à température ambiante. Cette caractéristique provient de l'effet attracteur d'électrons du noyau pyridine qui stabilise la fonction diazo de façon remarquable, comme le faisait le groupement nitro aryle des molécules de troisième génération.
2) une bien plus grande solubilité grâce la présence d'un atome d'azote sur le noyau pyridine.
3) une réactivité sur les acides nucléiques supérieure aux méthodes de marquage commerciales des acides nucléiques.

La présente invention est une amélioration conséquente des molécules existantes. En effet les molécules de première et de deuxième générations ont les inconvénients d'être instables chimiquement, même si ce point a déjà été amélioré par rapport à ce qui existait avant. Ainsi le marquage reste performant car les résultats obtenus sont très bons même après stockage à +4°C pendant une année. De plus, leur synthèse reste relativement complexe. Les molécules de troisième génération sont beaucoup plus stables et plus faciles à synthétiser ce qui présente des avantages considérables en terme de date de péremption des kits contenant ces molécules et d'industrialisation des synthèses.

Les molécules de première et de deuxième générations sont stables fonctionnellement sur une année si elles sont conservées à basse température en solvant organique anhydre. Les molécules de troisième génération sont beaucoup plus stables fonctionnellement et chimiquement que ce soit en milieu liquide ou sous forme sèche. Elles sont donc manipulables en milieu aqueux après avoir été conservées à sec, par séchage ou lyophilisation par exemple, sur une durée beaucoup plus longue (entre 10 et 100 fois plus), ce qui n'est pas le cas des molécules de première ou de deuxième génération qui sont instables pendant l'étape de lyophilisation.

Cette valorisation industrielle des molécules de troisième génération est particulièrement importante dans des dispositifs intégrés ou des microsystèmes, où la chimie embarquée se doit d'être performante et robuste sans devoir incriminer en cas de problème la stabilité de certains réactifs.

Enfin lesdites molécules de première et deuxième générations sont peu solubles dans une solution aqueuse, ce qui est gênant pour l'utilisateur qui doit, avant d'entamer une réaction, bien solubiliser ces molécules.

Toutefois même si ces molécules et procédés de marquage sont efficaces, la Demanderesse a réussi à trouver avec sa troisième génération de nouvelles molécules, et de nouveaux procédés de marquage de telles molécules qui améliorent encore l'efficacité du marquage, apportant une solution au problème de la stabilité des molécules de première et de deuxième générations.

Toutefois quelle que soit la génération, une à trois, de ces molécules, un autre problème réside dans le manque de solubilité de celles-ci. Ainsi deux nouvelles fonctionnalités moléculaires ont été associées pour créer ces nouveaux réactifs. Elles sont définies de la manière suivante :
- la fonction diazométhyle possède en *alpha* un cycle pyridine, pouvant être lui-même substitué une ou plusieurs fois par un ou plusieurs groupements en position *meta*, *para* ou *ortho.*
- la fonction diazo peut :
   - à l'instar des molécules de troisième génération, posséder en *alpha'* le groupement permettant la détection ; ce groupement peut être la biotine ou tout autre groupement détectable.
   - ne pas être porteuse du groupement détectable, qui comme les molécules de première et de deuxième générations, est porté par le cycle, c'est-à-dire le cycle pyridine, dans le cas présent.
Il est à noter que les molécules de quatrième génération sont aussi stables et réactives que les molécules de troisième génération, mais que leur solubilité est améliorée du fait de la présence d'un noyau pyridine qui stabilise également la fonction diazométhyle du fait de son effet attracteur.

Les définitions des « structure multimérique », « marqueur détectable », « systèmes indirects », « fluorophores » et autres marqueurs d'intérêt, « conjugaison », « molécule biologique », « acide nucléique », « technique d'amplification enzymatique », « solution sensiblement aqueuse », « solution homogène », « support solide », « étape de purification » sont données dans la demande de brevet WO-A-2005/092910, le lecteur est invité à s'y référer en cas de besoin.

De même, les techniques de :
- la chimie de greffage, et
- l'introduction de phosphate à l'extrémité 3' ou 5' des acides nucléiques,
sont également décrites dans cette demande de brevet ci-dessus et le lecteur peut retrouver dans ce document toutes informations nécessaires à la complète compréhension de la présente invention.

De plus la fonction diazométhyle portée par ces molécules de troisième génération, à l'instar des molécules de première et deuxième générations, permet de greffer de manière covalente les acides nucléiques sur le support. Le greffage est simple, la liaison est stable, par rapport à l'adsorption notamment, et permet de réaliser un couplage de l'acide nucléique sur le support solide, ce qui facilite d'autant les étapes d'hybridation ultérieures en diminuant l'encombrement stérique.

Cette nouvelle famille de molécules, dite de quatrième génération, est appelée Pyridine Diazo Cétone Biotine (PyDCB) ou Pyridine Diazo Biotine (PyDB) et est représentée par le réactif de marquage relativement stable à la température respectivement de formule (C) pour la PyDCB et de formule (D) pour la PyDB (voir ci-dessous).

Selon un premier mode de réalisation, la présente invention propose un réactif de marquage de formule (C) : 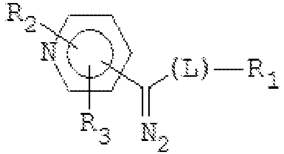 dans laquelle :
- R1 représente un marqueur détectable capable de générer directement ou indirectement un signal détectable et sélectionné parmi :
   - les enzymes qui produisent un signal par colorimétrie, fluorescence ou luminescence,
   - les chromophores,
   - les groupements à densité électronique détectable par microscopie électronique ou par leur propriété électrique,
   - les groupements détectables électro-chimiquement,
   - les dérivés d'un complexe de fer,
   - les molécules radioactives,
   - les couples ligand/anti-ligands choisis parmi les couples suivants : biotine/streptavidine, haptène/anticorps,antigène/ anticorps, peptide/anticorps, sucre/lectine, polynucléotide/ complémentaire du nucléotide,
   - les composés fluorescents de poids moléculaire inférieur à 1000g/ mole,
   - les haptènes de poids moléculaire inférieur à 1000g/mole,
   - les fluorophores,
- R2 et R3 représentent indépendamment l'un de l'autre : H, CONHR, COOR avec R correspondant à un groupe hydrocarboné aliphatique saturé comprenant de un à six atomes de carbone ou à un groupe aromatique monocyclique, et
- L est un bras de liaison comportant un enchaînement linéaire d'au moins deux liaisons covalentes comprenant un motif -(0-CH2 -CH2)-, répété de 1 à 20 fois.

Selon un second mode de réalisation, la présente invention propose un réactif de marquage de formule (D) : 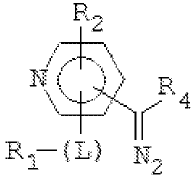 dans laquelle :
- R1 représente un marqueur détectable capable de générer directement ou indirectement un signal détectable et sélectionné parmi :
   - les enzymes qui produisent un signal par colorimétrie, fluorescence ou luminescence,
   - les chromophores,
   - les groupements à densité électronique détectable par microscopie électronique ou par leur propriété électrique,
   - les groupements détectables électro-chimiquement,
   - les dérivés d'un complexe de fer,
   - les molécules radioactives,- les couples ligand/anti-ligands choisis parmi les couples suivants : biotine/streptavidine, haptène/ anticorps,antigène/anticorps, peptide/anticorps, sucre/lectine, polynucléotide/complémentaire du nucléotide,
   - les composés fluorescents de poids moléculaire inférieur à 1000g/ mole,
   - les haptènes de poids moléculaire inférieur à 1000g/mole,
   - les fluorophores,
- R2 représente : H, CONHR, COOR avec R correspondant à un groupe hydrocarboné aliphatique saturé comprenant de un à six atomes de carbone ou à un groupe aromatique monocyclique,
- R4 représente : H, un groupe hydrocarboné aliphatique saturé comprenant de un à six atomes de carbone ou un groupe aromatique monocyclique, et
- L est un bras de liaison comportant un enchaînement linéaire d'au moins deux liaisons covalentes comprenant un motif -(O-CH2-CH2)-, répété de 1 à 20 fois.

L'implantation du bras portant à la fois le marqueur R₁ et la fonction diazométhyle (formule C) est en position para, *meta* ou *ortho* par rapport à la position d'implantation de l'atome d'azote du noyau pyridine. Préférentiellement ce bras est en position para par rapport à la position d'implantation de l'azote du noyau pyridine.

L'implantation du bras portant le marqueur R₁ ou l'implantation du bras portant la fonction diazométhyle (Formule D) sont, indépendamment l'une de l'autre, en position *para, meta* ou *ortho* par rapport à la position d'implantation de l'atome d'azote du noyau pyridine. Préférentiellement le bras portant la fonction diazométhyle est en position *para* par rapport à la position de l'atome d'azote du noyau pyridine, alors que ledit bras portant le marqueur R₁ est en position *ortho* par rapport à la position dudit atome d'azote du noyau pyridine.

De manière préférentielle, R₂ et/ou R₃ sont en position *meta* par rapport à la fonction diazo. Cette position permet la conjugaison de la fonction diazométhyle avec un substituant R₂ et/ou R₃.

Par groupe alkyle on entend au sens de la présente invention : un groupe hydrocarboné aliphatique saturé comprenant de un à six atomes de carbone (avantageusement un à deux, un à trois, un à quatre atomes de carbone) linéaire ou, quand la chaîne alkyle comprend au moins trois atomes de carbone, ramifié ou cyclique. A titre d'exemples, on peu citer les groupes méthyle, éthyle, propyle, isopropyle, butyle, iso-butyle, tertio-butyle, méthyl-cyclopropyle, pentyle, 2'2-diméthylepropyle, hexyle, cycloproyle, cyclobutyle, cyclopentyle ou cyclohexyle.

Au sens de la présente invention, on entend par groupe aryle un groupe aromatique monocyclique, par exemple un groupe phényle.

Selon une troisième variante de réalisation, la présente invention propose un réactif de marquage de formule (E) : 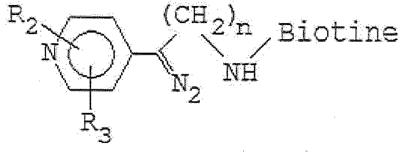 dans laquelle :
- R2 et R3 représentent indépendamment l'un de l'autre : H, CONHR, COOR avec R correspondant à un groupe hydrocarboné aliphatique saturé comprenant de un à six atomes de carbone ou à un groupe aromatique monocyclique, et
- n est un nombre entier compris entre 1 et 12, préférentiellement entre 1 et 6.

Selon une quatrième variante de réalisation, la présente invention propose un réactif de marquage de formule (F) : 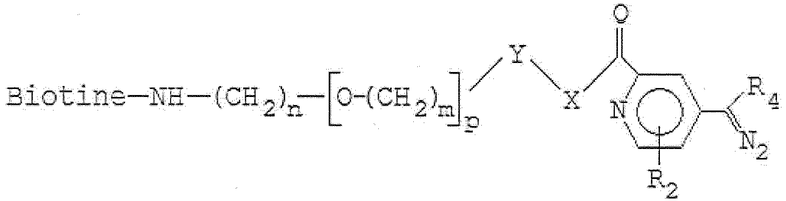 dans laquelle :
- R2 représente : H, CONHR, COOR avec R correspondant à un groupe hydrocarboné aliphatique saturé comprenant de un à six atomes de carbone ou à un groupe aromatique monocyclique,
- R4 représente : H, un groupe hydrocarboné aliphatique saturé comprenant de un à six atomes de carbone ou un groupe aromatique monocyclique,
- -Y-X- représente -CH₂NH-, -CONH-, -NHCO-, -CH₂O- ou -CH₂-S-, et
- m, n et p sont chacun, indépendamment les uns des autres, un nombre entier compris entre 1 et 12, préférentiellement entre 1 et 6.

Selon une variante de la troisième variante de réalisation, la présente invention propose un réactif de marquage de formule (G) :

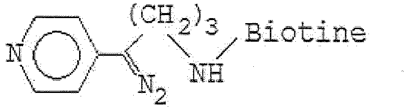

Selon une variante de la quatrième variante de réalisation, la présente invention propose un réactif de marquage de formule (H) :

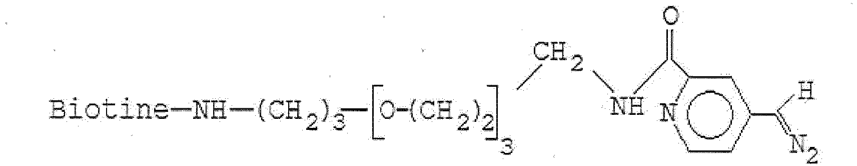

Quelque soit le mode de réalisation, le réactif, selon l'invention exposée ci-avant, comporte préférentiellement un radical R₁, qui est constitué par un résidu D-Biotine de formule (I) :

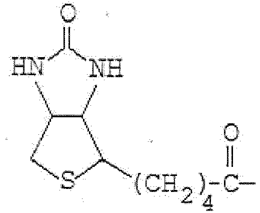

La présente invention concerne également un procédé de synthèse d'un réactif de marquage, tel qu'exposé ci-dessus, comprenant les étapes suivantes (molécule du type PyDCB) :
a) un dérivé d'acide carboxylique aromatique est mis en réaction avec l'énolate d'une lactone (réaction de type Claisen) pour former un précurseur cyclique, lequel précurseur cyclique est ensuite ouvert avec un acide halogéné pour former une cétone aromatique halogénée,
c) la fonction carbonyle de la cétone aromatique halogénée est protégée par un groupement protecteur pour former un précurseur protégé,
d) lequel précurseur protégé est soumis à une réaction d'amination (de type Gabriel) pour former un précurseur aminé,
e) lequel précurseur aminé est déprotégé pour libérer la fonction amine, ladite fonction amine étant mise en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin
g) le précurseur marqué et carbonylé est transformé en un réactif de marquage, tel que décrit ci-dessus, par une transformation de la fonction carbonyle en une fonction diazo.

En ce qui concerne la synthèse de molécules du type PyDB, les étapes sont les suivantes :
a) un acide aromatique di-carboxylique est estérifié pour former un diester,
b) ce diester est substitué régio-sélectivement avec un composé dérivé de l'éthylène glycol aminé mono protégé pour former un précurseur protégé sur la fonction carboxylique et sur la fonction amine,
c) lequel précurseur est ensuite réduit en alcool et oxydé en aldéhyde,
d) l'aldéhyde, synthétisé à l'étape c), est soumis à un traitement acide afin de, concomitamment protéger cette fonction tout en déprotégeant la fonction amine, pour conduire à un acétal aminé,
e) lequel acétal aminé est mis en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin,
g) le précurseur marqué et carbonylé est transformé en un réactif de marquage, tel que décrit ci-dessus, par une transformation de la fonction carbonyle en une fonction diazo selon une réaction du type de Bamford Stevens.

La présente invention concerne encore un procédé pour le marquage d'une molécule biologique, en particulier un acide nucléique, comprenant la mise en contact en solution homogène, dans un tampon sensiblement aqueux, d'une molécule biologique et d'un réactif.

L'invention propose également une molécule marquée susceptible d'être obtenue par le procédé de marquage d'une molécule biologique ci-dessus exposée.

La présente invention concerne aussi un procédé de marquage et de fragmentation d'un acide nucléique simple ou double brin comprenant les étapes suivantes :
- fragmenter l'acide nucléique,
- attacher un marqueur sur au moins un des fragments par l'intermédiaire d'un réactif de marquage choisi parmi les réactifs,
ledit réactif se couplant de manière covalente et majoritaire sur au moins un phosphate dudit fragment. Une telle technique de fixation sur le phosphate est, par exemple, représentée sur la figure 1.

Selon une première variante de réalisation, le procédé est caractérisé par le fait que la fragmentation et le marquage sont effectués en deux étapes.

Selon une seconde variante de réalisation, le procédé est caractérisé par le fait que la fragmentation et le marquage sont effectués en une étape.

Quelle que soit la variante de réalisation, le procédé est caractérisé par le fait que le marquage s'effectue en solution homogène sensiblement aqueuse.

Quelle que soit la variante de réalisation, le procédé est caractérisé par le fait que la fragmentation s'effectue par voie enzymatique, physique ou chimique.

Les procédés ci-dessus décrits, permettent à l'invention de revendiquer également un acide nucléique marqué susceptible d'être obtenu par ces procédés.

La présente invention concerne encore un kit de détection d'un acide nucléique cible comprenant un acide nucléique marqué, tel que décrit plus haut.

La présente invention concerne également un support solide sur lequel est fixé un réactif, tel que décrit plus haut.

La présente invention concerne enfin un procédé de capture d'acides nucléiques comprenant les étapes suivantes :
- on dispose d'un support solide sur lequel est fixé directement ou indirectement au moins une molécule biologique, tel que décrit ci-dessus, ou un acide nucléique, tel que décrit plus haut, la molécule biologique ou l'acide nucléique comprenant une fonction diazométhyle,
- on met en contact un échantillon biologique susceptible de contenir des acides nucléiques libres, et
- on lave le support solide où la (ou les) molécule (s) sont fixée(s) de manière covalente au moins à un acide nucléique.

Ces molécules de quatrième génération DCB comportent un bras espaceur L, appelé linker, et un marqueur R₁ qui pourrait être constitué par un groupement détectable, tel que la biotine, un haptène, un fluorophore, un groupement fluorescent, un groupement luminescent, etc.

L est un bras de liaison comportant un motif -(O-CH₂-CH₂)-, répété de 1 à 20 fois, préférentiellement de 1 à 12 fois, et encore plus préférentiellement de 2 à 6 fois.

Les exemples et figures ci-joints représentent des modes particuliers de réalisation et ne peuvent pas être considérés comme limitant la portée de la présente invention.
La Figure 1 représente un schéma réactionnel montrant le marquage d'un acide nucléique simple brin, qu'il soit sous forme d'ARN ou d'ADN par une molécule selon l'invention.
La Figure 2 est un récapitulatif des molécules utilisées dans la présente demande d'invention.

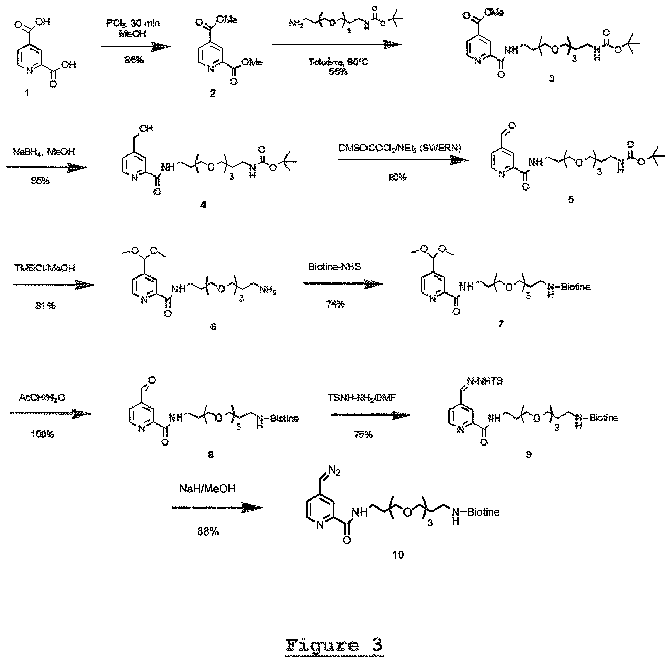

La Figure 3 représente un schéma réactionnel du procédé de synthèse d'une molécule de PyDB (molécule 10)
La Figure 4 représente un schéma réactionnel du procédé de synthèse d'une molécule de PyDCB (molécule 19)
La Figure 5 représente la stabilité comparée en milieu liquide des molécules PyDB et PyDCB par rapport à une molécule de [Bio-EG3]2-PDAM de deuxième génération (ci-après appelée BBP) et décrite dans la demande WO-A-02/090319.
La Figure 6 représente la stabilité comparée sous forme sèche des deux molécules de quatrième génération PyDB et PyDCB par rapport à une molécule de deuxième génération BBP.
La Figure 7 représente les résultats de marquage d'un amplicon d'ARN après purification à l'aide des marqueurs BBP et PyDB chacun des marqueurs étant à une concentration de 45 mM.
La Figure 8 représente les résultats de marquage d'un amplicon d'ARN sans purification à l'aide des marqueurs BBP et PyDB, chacun des marqueurs étant à une concentration de 2 mM et en présence 3 mM HCL.
La Figure 9 représente la stabilité du marquage d'un amplicon d'ARN marqué par les marqueurs PyDB ou BBP, à une concentration de 2,5 mM, sur 24 heures d'hybridation.
La Figure 10 représente la comparaison de l'efficacité de marquage d'un amplicon d'ARN en fonction de la technique de marquage employée, soit ULS (Universal Labelling System) de Kreatech (Amsterdam, Pays-bas), soit selon la présente invention.
La Figure 11 représente la solubilité comparative en milieu aqueux de deux molécules de troisième génération (*meta* Nitro DCB et *para* Nitro DCB) par rapport à deux molécules de quatrième génération (PyDB et PyDCB), établie par HPLC.

Dans les figures et dans la description, les termes « Fluorescence relative » et « RFU » sont identiques.

Dans les exemples écrits ci-après, les abréviations suivantes seront utilisées :
- Ar : aromatique,
- s : singulet,
- se : singulet élargi,
- d : doublet,
- dd : doublet dédoublé,
- t : triplet,
- q : quadruplet
- qu: quintuplet,
- m : massif,
- M : multiplet,
- HPLC : chromatographie liquide haute performance,
- CCM : chromatographie sur couche mince,
- RMN : résonance magnétique nucléaire,
- Rdt : rendement,
- Eq : équivalents
- Rf ou TR : temps de rétention,
- DMSO-d6 : diméthylsulfoxide deutéré,
- DMCF : diméthyl cyclohexylamonium formiate,
- CDCl₃ : Chloroforme deutéré, et
- DMF : diméthylformamide,
- DCM : Dichlorométhane,
- MeOH : Méthanol,
- ACN : Acétonitrile,
- AcOET : Acétate d'éthyle,
- Eau MilliQ : Eau ultrapure (Millipore, Molsheim, France),
- DMAC : Diméthyle amino cinnamaldéhyde,
- Si60 : Silica gel 60 FLUKA (40-63 µm),
- UV : ultraviolet.

### Les conditions générales pour l'analyse et la synthèse des composés chimiques décrits (exemples 1, 2, 3, 4 et 9) sont :

### Matériel :

Conditions HPLC (Chaîne HPLC WATERS Alliance 2795, Détecteur à barette de diodes PDA 996, logiciel Empower version 2 et colonne WATERS XTerra MS C18 4,6 x 30 2,5 µM) avec un débit de 1 ml/minute à 30°C (détection à 260 nm).

### Méthode HPLC basique :

| | |
|---|---|
| Eluant A : | eau milliQ |
| Eluant B : | ACN |
| Eluant C : | ammoniaque à 500 mM pH 12 |

Soit un gradient linéaire de 1 à 64% d'acétonitrile (5 mM constants d'ammoniaque à pH=9) en 20 minutes.

### Méthode HPLC Neutre :

| | |
|---|---|
| éluant A : | eau milliQ |
| éluant C : | ACN |
| éluant D : | Solution à 500mM de formiate d'ammonium pH 7 |

Soit un gradient linéaire de 1 à 64% de C dans un solution à 10mM en formiate d'ammonium en 20 minutes.

### Synthèse :

Les analyses par chromatographie sur couche mince ont été réalisées sur des plaques de silice ALUGRAM® MACHEREY-NAGEL SIL G/UV₂₅₄ 4 x 8 cm (Duren, Allemagne) avec une détection UV à 254 nm ou au DMAC pour les produits biotinylés.

La purification des produits a été effectuée par chromatographie sur gel de silice Silica gel 60 FLUKA (40-63 µm). Les conditions de séparation par chromatographie « flash » (sous pression argon) respectent strictement les conditions décrites par Clark Still *et al* (Clark Still, W. ; Kahn, M. ; Mitra, A. J. Org. Chem. 1978, 43, 2923-2925) soit une hauteur fixe de silice de 15 cm poussée à un débit de 5 cm/minutes, le diamètre de la colonne dépendant de la quantité et des Rf des produits à purifier.

Pour l'exemple 1, les spectres RMN ont été enregistrés sur un spectromètre Brüker 200 MHz. Les déplacements chimiques (δ) sont donnés en ppm par rapport au pic du solvant pris comme référence interne (CDCl₃ : 7,24 ppm ; DMSO-d6 : 2,49 ppm ; D₂O : 4,80 ppm à 25°C). Les spectres sont décrits avec les abréviations ci-dessus : s, d, t, q, qu, m et M. Les constantes de couplage (J) sont exprimées en hertz (Hz)

Pour l'exemple 2, les spectres de RMN ¹H et ¹³C ont été réalisés sur des spectromètres Bruker AC 200 ou Bruker DPX 300 à température ambiante. Les déplacements chimiques (δ) sont exprimés en ppm par rapport au tétraméthylsilane et calibrés par rapport au CHCl₃ (δ_{H} = 7,27, δ_{C} = 77,0) ou au DMSO (δ_{H} = 2,52, δ_{C} = 40,0). Les constantes de couplage (J) sont exprimées en hertz (Hz). La forme des signaux est abrégée de la façon suivante : se et dd.

### Spectres de masse :

Les spectres de masse (SM) ont été obtenus avec un appareil LCQ-ion trap (Thermofinnigan, San Jose, CA, USA) par des méthodes d'ionisation Electrospray en mode positif par infusion à travers un tube de silice 2 à 10 µL/minute. Les principaux solvants utilisés sont le DCM et le MeOH.

### Exemple 1 : Synthèse du Pyridine Diazo Biotine (PyDB) :

### Objectif :

Démontrer la faisabilité de la synthèse d'une molécule contenant un noyau pyridine en *alpha* d'une fonction diazométhyle, et un groupement de reconnaissance sur le cycle aromatique, selon le schéma réactionnel ci-dessous exposé :

### Mode Opératoire :

### → Pyridine-2,4-dicarboxylate de diméthyle (2)

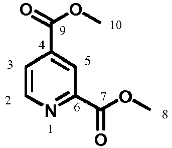

Dans un ballon de 500 mL sont introduits le PCl₅ (39 g ; 0,186 mol ; 3 eq) et l'acide pyridine-2,4-dicarboxylique (11,5 g; 6,2×10⁻² mol). Le mélange est agité à température ambiante pendant 45 minutes : il devient un liquide vert. On le dilue ensuite avec du dichlorométhane (80 mL) puis on ajoute du méthanol (20 mL) avec précaution. Le tout est versé dans de l'eau H₂O (300 mL) avec 60 g de NaHCO₃, la phase aqueuse est lavée avec du dichlorométhane (4 × 50 mL) et la phase organique est lavée avec du Na₂CO₃ 1M (2 × 50mL) . Le produit brut est purifié par flash chromatographie (éluant : AcOEt/Cyclohexane 75:25).
Solide : m = 11,71 g
Rendement : 96%
Stockage : température ambiante
**RMN ¹H (200MHz, CDCl₃) :** 4.03 (s, 3H, -OMe) ; 4.09 (s, 3H, - OMe) ; 8.07 (dd, J=6Hz, 1H, *H₃*) ; 8.68 (s, 1H, *H₅*) ; 8.91 (d, J=4,6Hz, 1H, *H₂*)
**RMN ¹³C (75MHz, CDCl₃) :** 53.28 (C₈, C₁₀) ; 124.49 (C₅) ; 126.29 (C₃) ; 138.64 (C₄) ; 149.06 (C₆) ; 150.92 (C₂) ; 162.45 (C₇) ; 164.96 (C₉)

### → 2-{N-[13-(t-Butoxycarbonylamino)-4,7,10-trioxatridécyl] aminocarbonyl}pyridine-4-carboxylate de méthyle (3)

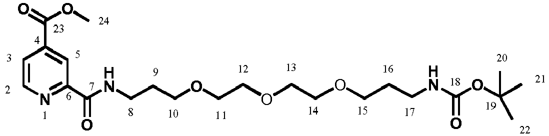

Dans un ballon de 25 mL, on introduit le diester 2 (2,25g ; 1,1×10⁻² mol) dans le toluène (3 mL). La Boc-diamine (3,7 g ; 1,2×10⁻² mol ; 1,1 eq) est ajouté puis le mélange est agité à 90°C pendant 20 heures. Le toluène est évaporé puis le mélange brut est purifié par flash chromatographie (gradient d'éluant : dichlorométhane/acétone 10, 20 et 30%).
Huile transparente : m = 2,9 g
Rendemendt : 55%
Stockage : température ambiante
**RMN ¹H (300MHz, CDCl₃) :** 1.42 (s, 9H, -Boc) ; 1.74 (q, 2H, H₉) ; 1.92 (q, 2H, *H*₁₆) ; 3.21 (q, 2H, *H*₈) ; 3.61 (m, 16H, H₁₀-H₁₁-H₁₂-H₁₃-H₁₄-H₁₅-H₁₇) ; 3,97 (s, 3H, -O*Me*) ; 5.01 (m, 1H, N*-H*) ; 7.96 (d, 1H, H₅) ; 8.35 (s+d, 2H, H₃+H₂) ; 8.69 (m, 1H, N*-H*)
**RMN ¹³C (75MHz, CDCl₃) :** 28.63 (C₂₀-C₂₁-C₂₂) ; 29.30 (C₁₆) ; 29.74 (C₉) ; 37.55 (C₁₇) ; 38.85 (C₈) ; 53.02 (C₂₄) ; 69.69 (C₁₀-C₁₅-C₁₁-C₁₄) ; 70.78 (C₁₂-C₁₃) ; 79.97 (C₁₉) ; 121.60 (C₅) ; 125.52 (C₃) ; 139.08 (C₄) ; 149.23 (C₂) ; 151.76 (C₆) ; 156.27 (C₁₈) ; 163.65 (C₇) ; 165.60 (C₂₃)

### → 4-(Hydroxyméthyl)pyridine-2-N-[13-(t-butoxycarbonylamino)-4,7,10-trioxatridécyl]-carboxamide (4)

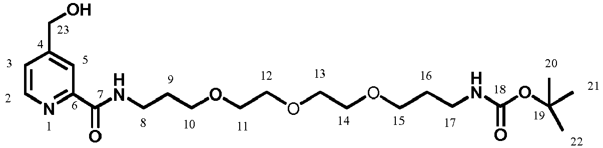

Dans un ballon de 250 mL on dissout le monoester (2,73 g ; 5,4×10⁻³ mol) dans le MeOH (20 mL). Le mélange est refroidi à l'aide d'un bain de glace, NaBH₄ (1,32 g; 3,5×10⁻² mol ; 6,5 eq) est ajouté petit à petit. La mixture est agitée pendant 30 minutes à température ambiante.

Le mélange est dilué dans H₂O (100 mL) et neutralisé avec NH₄Cl saturé puis extrait avec dichlorométhane (4 × 100 mL). Les phases organiques sont rassemblées, évaporées et séchées sous vide du dessiccateur pendant une nuit.
Huile transparente : m = 2,44 g
Rendement : 95%
Stockage : température ambiante
**RMN ¹H (200MHz, CDCl₃) :** 1.42 (s, 9H, -Boc) ; 1.75 (q, 2H, H₉) ; 1.91 (q, 2H, *H*₁₆) ; 3.02 (m, 1H, O*H*) ; 3.19 (q, 2H, *H*₈) ; 3.59 (m, 16H, H₁₀-H₁₁-H₁₂-H₁₃-H₁₄-H₁₅-H₁₇) ; 4.79 (d, 2H, H₂₃) ; 5.03 (m, 1H, N-*H)* ; 7.47 (d, 1H, H₃) ; 8.15 (s, 1H, H₅) ; 8.39 (m, 1H, *N-H)* ; 8.51 (d, J=5Hz, 1H, H₂)
**RMN ¹³C (75MHz, CDCl₃)** : 28.77 (C₂₀-C₂₁-C₂₂) ; 29.26 (C₁₆) ; 30.07 (C₉) ; 37.68 (C₁₇) ; 39.01 (C₈) ; 63.70 (C₂₃) ; 69.42 (C₁₀-C₁₅) ; 70.39 (C₁₁-C₁₄-C₁₂-C₁₃) ; 79.96 (C₁₉) ; 119.95 (C₅) ; 123.87 (C₃) ; 148.44 (C₂) ; 150.03 (C₆) ; 143.34 (C₄) ; 156.42 (C₁₈) ; 164.96 (C₇)

### → 4-(Formyl)pyridine-2-N-[13-(t-butoxycarbonylamino)-4,7,10-trioxatridécyl]carboxamide (5)

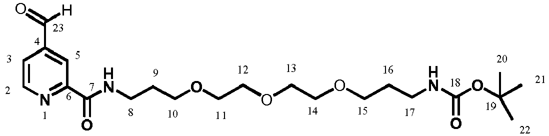

A une solution de DMSO (2,0 mL, 28,2 mmol) dans du dichlorométhane (8 mL), on ajoute (COCl)₂ (1,2 mL, 13,7 mmol) en solution dans dichlorométhane (5 mL) à -70°C sous argon. On laisse agiter pendant 30 minutes à -70°C puis on ajoute goutte à goutte l'alcool **4** (1,92 g, 4,2 mmol) en solution dans le dichlorométhane (20 mL). On laisse agiter pendant 30 minutes à une température de -70°C, on ajoute NEt₃ (6,5 mL, 47 mmol). Le mélange réactionnel est agité à 0°C pendant 3 h, ensuite versé dans de l'eau (70 mL). La phase aqueuse est extraite avec ledit dichlorométhane, lavée (NaHCO₃), séchée sur MgSO₄ et évaporée en une huile visqueuse. Il y a alors un dégagement de Me₂S, pour lequel il faut utiliser de l'eau de javel afin de piéger et de laver les vaisselles contaminées. Le produit brut est purifié par flash chromatographie (éluant : AcOEt/acétone 85:15).
Huile transparente :
   m = 1,46 g
Rendement : 75 %
**RMN ¹H (300MHz, CDCl₃) :** 1.42 (s, 9H, -Boc) ; 1.76 (q, J=6Hz, 2H, H₉) ; 1.95 (q, J=6Hz, 2H, *H*₁₆) ; 3.22 (q, 2H, *H*₈) ; 3.57 (m, 16H, H₁₀-H₁₁-H₁₂-H₁₃-H₁₄-H₁₅-H₁₇) ; 4.98 (m, 1H, *N-H)* ; 7.85 (d, 1H, H₃) ; 8.59 (s, 1H, H₅) ; 8.77 (d, 1H, H₂) ; 10.15 (s, 1H, Hₐₗ)
**RMN ¹³C (75MHz, CDCl₃) :** 29.59 (C₂₀-C₂₁-C₂₂) ; 30.03 (C₉-C₁₆) ; 38.11 (C₈-C₁₇) ; 70.11 (C₁₀-C₁₅) ; 70.99 (C₁₁-C₁₄-C₁₂-C₁₃) ; 79.50 (C₁₉) ; 122.27 (C₅) ; 123.68 (C₃) ; 143.22 (C₄) ; 149.80 (C₂) ; 152.61 (C₆) ; 156.35 (C₁₈) ; 163.62 (C₇) ; 191.32 (C₂₃)

### → 4-(Diméthoxyméthyl)pyridine-2-N-(13-amino-4,7,10-trioxatridé cyl)-carboxamide (6)

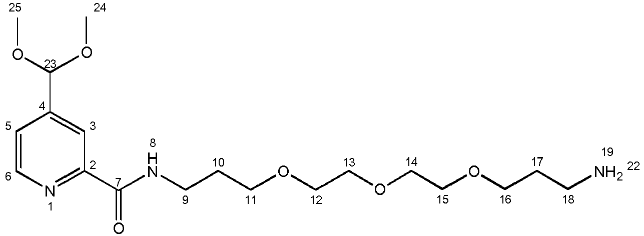

On dissout l'aldéhyde 5 (1.4 g, 3.1 mmol, 1 eq) dans 10 mL de méthanol. On ajoute le TMSCl (1mL, 7.9 mmol, 2.5 eq) et le mélange est agité toute la nuit. On ajoute une solution d'hydroxyde de sodium 1 M (40 mL)) et le produit est extrait avec le dichlorométhane, les phases organiques sont séchées en présence de carbonate de sodium et le solvant est évaporé sous vide.
M = 997 mg
Rendement : 81%
**¹H NMR (300MHz, CDCl₃) :** 8.54 (d, 1H, H₆), 8.35 (br s, 1H, H₈), 8.24 (s, 1H, H₃), 7.52 (d, 1H, H₅), 5.42 (s, 1H, H₂₃), 3.71-3.53 (m, 15H, H₉+H₁₁₋₁₆), 3.33 (s, 6H, H₂₄, ₂₅), 2.80 (t, 2H, H₁₈), 1.91 (quint, 2H, H₁₀), 1.75 (quint, 2H, H₁₇).

### → Acetal-Biotine (7)

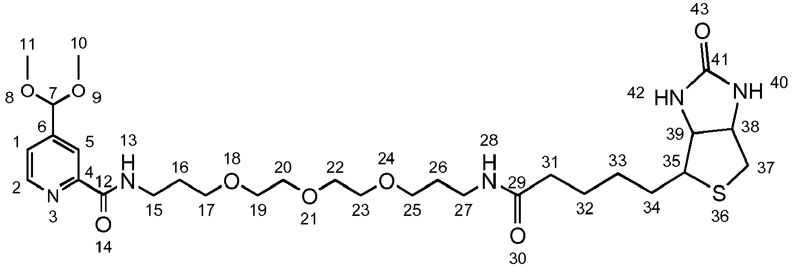

L'acétal (6) (1.54 g, 3.9 mmol) est dissous dans 30 ml de dichlorométhane. On ajoute la biotine (1.36 g, 4.2mmol, 1.1 eq) et le mélange est agité pendant deux heures. La solution est extraite trois fois avec 50 mL de carbonate de sodium 1 M. Les phases aqueuses sont lavées avec 50 mL de dichlorométhane. La phase organique est séchée avec sulfate de magnésium et le solvant est évaporé. Le brut est purifié par flash chromatographie: SiO₂ Φ= 50 mm, 15 cm. Eluant : 700 mL 10% méthanol/dichlorométhane et puis 1 L 15% méthanol/ dichlorométhane. Ensuite le solvant est évaporé, et le résidu huileux est lavé avec de l'éther. Une poudre blanche est obtenue.
m = 1.78 g
Rendement : 74%
**¹H NMR (300MHz dual, CDCl₃) :** 8.55 (d, 1H, H₂), 8.35 (t, 1H, H₁₃), 8.26 (s, 1H, H₅), 7.55-7.53 (m, 1H, H₁), 6.7 (broad s, 1H, H₄₂), 6.2 (broad s, 1H, H₄₀), 5.44 (s, 1H, H₇), 4.4- 4.6 (m, 1H, H₃₈), 4.2- 4.4 (m, 1H, H₃₉), 3.71- 3.54 (m, 16H, H₁₅, H₁₇₋₂₅, H₂₇), 3.34 (s, 6H, H₁₀₋₁₁), 3.2-3.1 (m, 1H, H₃₅), 3-2.8 (m, 1H, H₃₇), 3.8-3.7 (m, 1H, H_{37'}), 2.19 (t, 2H, H₃₁), 1.92 (quint, 2H, H₁₆), 1.8-1.3 (m, 8H, H₂₆, H₃₂₋₃₄).

### → Aldéhyde (8)

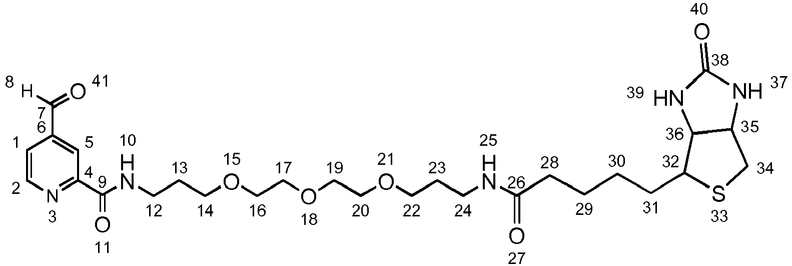

L'acétal (7) (1.76 g, 2.8 mmol) est dissous dans une solution d'acide acétique/eau 80:20 (20 mL) et la mélange est agité à 60°C pendant 24 heures sous argon. La solution est évaporée et le résidu huileux est lavé avec de l'éther jusqu'à ce qu'une poudre blanche soit obtenue.
M = 1.83 g
Rendement : environ 100%
**¹H NMR (300MHz dual, DMSO-d6) :** 10.17 (s, 1H, H₈), 8.92 (s+d, 2H, H_{10,2}), 8.42 (s, 1H, H₅), 7.99 (d, 1H, H₁), 7.73 (t, 1H, H₂₅), 6.4-6.3 (s+ broad s, 2H, H_{39,37}), 4.3-4.1 (m+m, 2H, H_{35,36}), 3.53-3.28 (m, 18H, H_{12,14-22,24}), 3.07 (m, 1H, H₃₂), 2.80 (d, 1H, H₃₄), 2.78 (d, 1H, H_{34'}), 2.2-1.00 (m, 12H, H_{13,28-31}).

### → Tosylhydrazone (9)

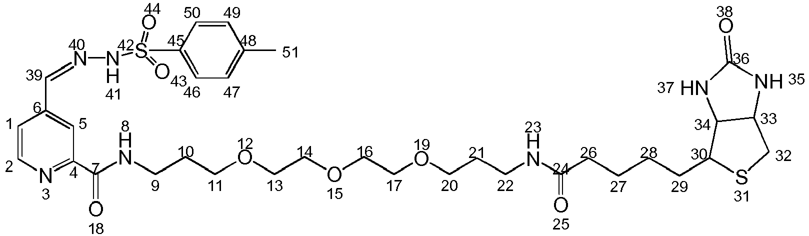

Une solution d'aldéhyde 8 (1.62 g, 2.79 mmol) dans du diméthylformamide (20 mL) est agitée à 130°C pendant 30 minutes sous argon. On ajoute la 4-méthylbenzène-sulfonhydrazide et le mélange est agité pendant une heure. On évapore le solvant et le brut est purifié par flash chromatographie: SiO₂, Φ= 30 mm, 15 cm, éluant : 10% méthanol/dichlorométhane. Le résidu huileux est lavé avec de l'éther jusqu'à ce qu'une poudre jaune soit obtenue.
M = 1.57 g
Rendement : 75%
**¹H NMR (300MHz dual, DMSO-d6) :** 12.0 (broad s, 1H, H₃₉), 8.83 (s, 1H, H₈), 8.63 (d, 1H, H₂), 8.13 (s, 1H, H₄₁), 7.99 (s, 1H, H₅), 7.77 (d, 2H, H₄₆, ₅₀), 7.69 (d, 1H, H₁), 7.42 (d, 2H, H_{47, 49}), 6.41 (s, 1H, H₃₇), 6.35 (s, 1H, H₃₅), 4.4-4.2 (m, 1H, H₃₄), 4.0-4.2 (m, 1H, H₃₃), 3.51- 3.33 (m, 16H, H₉, _{11-20, 22}), 3.1-3.0 (m, 2H, H₃₂), 2.8-2.7 (m, 1H, H₃₀), 2.36 (s, 3H, H₅₁), 2.03 (t, 2H, H₁₀), 1.77 (t, 2H, H₂₁), 1.6-1.2 (m, 8H, H₂₆₋₂₉).

### → Composé Diazo Final (10)

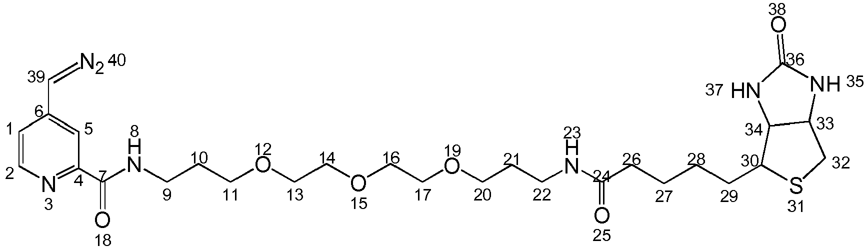

Dans un ballon de 25 mL, on introduit la tosylhydroazone 9 (200 mg, 0.27 mmol). On ajoute une solution d'hydride de sodium (60% en huile) (42.8 mg, 1.07 mmol, 4 eq) dans méthanol (10 mL) et on laisse agiter pendant 24 heures à température ambiante. Le produit est extrait avec 10 mL Na₂CO₃ 0.5 M et 4×10mL de dichlorométhane. Le résidu huileux est lavé avec de l'éther. Une orange poudre est obtenue.
M = 135 g
Rendement : 88%
**¹H NMR (200MHz dual, CDCl₃) :** 8.31-8.28 (br s+d, 2H, H₈,₂), 7.73 (d, 1H, H₅), 6.88 (dd, 1H, H₁), 6, 62 (t, 1H, H₂₃), 6.13 (s, 1H, H₃₇), 5.32 (s, 1H, H₃₅), 5.10 (s, 1H, H₃₉), 4.6- 4.4 (m, 1H, H₃₄), 4.4- 4.2 (m, 1H, H₃₃), 3.7- 3.5 (m, 18H, H_{9, 11-20, 22}), 3.13 (m, 1H, H₃₀), 2.88 (dd, 1H, H₃₂), 2.72 (d, 1H, H_{32'}), 2.2- 1.3 (m, 12H, H_{10, 21, 26-29}).

### Résultats et conclusions :

**Nous avons démontré que la synthèse d'un composé comportant une fonction diazo méthyle conjuguée à un noyau pyridine portant un groupement de reconnaissance (biotine) était réalisable avec un bon rendement.**

### Exemple 2 : Synthèse de la Pyridine Diazo Cétone Biotine (PyDCB) :

### Objectif :

Démontrer la faisabilité de la synthèse d'une molécule contenant un noyau pyridine en *alpha* d'une fonction diazo et un groupement de reconnaissance (Biotine) en *alpha* prime de la fonction diazo selon le schéma réactionnel ci dessous.

### Mode Opératoire :

### → Synthèse de la cétone bromée (12)

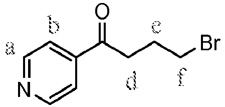

La γ-Butyrolactone (5,020 g/4,55 ml ; 32,23 mmol ; 1,0 eq) est dilué dans du THF (15 ml ; 3,3 v) puis refroidit à 0°C dans un bain de glace. L'hydrure de sodium (à 60% ; 3,018 g ; 75,42 mmol ; 2,27 eq) est ajouté par portion en l'espace de 5 minutes puis le bain de glace est retiré pendant 30 minutes. Le ballon est remis à 0°C puis l'isonicotinate d'éthyle (3,80 ml ; 49,84 mmol ; 1,5 eq) en solution dans du THF (245 ml ; 64,5 v) est coulé goutte à goutte. La réaction est agitée 18 heures à température ambiante. Le THF est éliminé par évaporation sous vide puis le résidu est repris avec 100 ml d'éther éthylique le produit attendu précipite, la suspension est essorée, puis séché au roto à 1 mbar pendant 20 minutes avec obtention d'un produit. Une partie du produit (4,721 g ; 24,7 mmol) est repris par de l'acide bromhydrique à 48% (38 ml soit 650 mM) dans l'eau puis portée à 110°C pendant 2 heures. Le mélange réactionnel est basifié jusqu'à pH 9 avec du Na₂CO₃ puis complété avec 200 ml d'eau et extrait deux fois avec 200 ml d'acétate d'éthyle. La phase organique est séchée sur du Na₂SO₄, filtrée sur coton et évaporée au rotavapor.
m = 3,096g
Rendement (sur les 2 étapes) : 21%
Méthode HPLC : neutre

### → Synthèse de la cétone bromée et protégé (13)

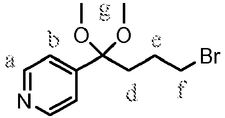

Dans un ballon de 500 ml, la cétone bromée (3,096 g ; 13,57 mmol ; 1,0 eq) est mise en solution dans du méthanol anhydre (165 ml ; 80 mM) auquel du chlorure de triméthylsilyl est ajouté (51,5 ml ; 407,2 mmol ; 30,0 eq) puis la réaction est mise sous argon avant de chauffer à 53°C (température inférieure au point d'ébullition des réactifs) pendant 18 heures. Le mélange réactionnel est évaporé à sec puis repris dans 50 ml d'eau et basifié à la soude 35% jusqu'à pH 9-10 puis complété à 200 ml et extrait avec deux fois 150 ml de dichlorométhane (également appelé de manière abrégée, DCM). La phase organique est séchée sur Na₂SO₄ filtrée et évaporée à sec sous pression réduite. Le résidu d'évaporation est repris avec 1 ml de DCM et déposé sur une colonne de gel de silice phase normal Si60 (h = 5 cm ; diam.= 15 cm ; éluant : DCM/MeOH: 95/5).
m = 1,506g
Rendement : 40%
Eluant CCM : DCM/MeOH: 95/5
Méthode HPLC : neutre
**RMN ¹H** (200 MHz, CDCl3) : δ = 1,4 à 1,7 (M ; 2H ; e) ; 3,0 à 3,25 (m; 8H ; f,g) ; 3,30 (t ;2H ; d) ; 7,42 (d ; 2H ; a) ; 8,65 (d ; 2H ; b).

### → Cétone protégée et aminée (Réaction de Gabriel) (15)

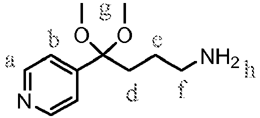

Dans un ballon de 100 ml, la cétone bromée (1,506 g ; 5,49 mmol ; 1,0 eq), le phtalimide de potassium (1,526 g ; 8,24 mmol ; 1,5 eq) et le DMF (55 ml 0,1 M) sont chargés puis sont chauffés 15 minutes à 155°C. Le solvant est évaporé à sec, puis le résidu d'évaporation est repris avec 200 ml d'une solution de soude à 100 mM et extrait avec deux fois 200 ml de DCM, la phase dichlorométhane est séchée sur Na₂SO₄ filtrée, et évaporée à sec pour donner le composé 14. Son intégralité (5,49 mmol ; 1,0 eq) est mise en réaction avec de l'hydrazine à 25% dans l'eau (21,35 ml ; 109,8 mmol ; 20 eq) et du méthanol (109 ml ; 50 mM), la réaction est agitée pendant 18 heures. Les solvants sont éliminés puis le résidu est repris dans 200 ml d'une solution de soude à 10 mM puis extrait avec trois fois 200 ml de DCM. La phase organique est séchée sur Na₂SO₄ filtrée et évaporée sous pression réduite puis l'huile obtenue est reprise par 1 ml de DCM et déposée sur une colonne de gel de silice phase normal Si60 (h = 15 cm ; diam. = 3 cm ; éluant : DCM/MeOH/NH₃ : 97,5/2,5/1).
m = 300 mg
Rendement : 26%
Eluant CCM : DCM/MeOH/NH₃ : 90/10/1
Méthode HPLC : neutre
**RMN ¹H** (phtalimide 14) (200 MHz, CDCl3) : δ = 1,2 à 1,3 (M ; 2H ; e) ; 1,88 (M; 2H ; d) ; 3,03 (t ; 6H ; g) ; 3,44 (dd ;2H ; f) ; 7,45 (d ; 2H ; a) ; 7,57 à 7,71 (m ; 4H ; i et j) ; 8,52 (d ; 2H ; b).
**RMN ¹H** (amine 15) (200 MHz, CDCl3) : δ = 1,0 à 1,2 (m ; 4H ; e et f) ; 1,88 (M; 2H ; d) ; 2,54 (t ; 2H ; h) ; 3,15 (t ; 6H ; g) ; 7,35 (d ; 2H ; a) ; 8,58 (d ; 2H ; b).

### → Synthèse de la biotine activée (20)

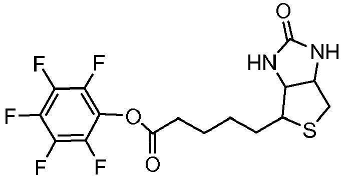

La biotine (5 g ; 23,1 mmol ; 1,0 eq) est mise en suspension dans le DMF anhydre (50 ml) et la pyridine (2,07 ml ; 25,4 mmol ; 1,1 eq). Après 5 minutes d'agitation, le pentafluorophenyl trifluoroacétate (PFP-TFA : 4,621 ml, soit 7,50 g ; 25,4 mmol ; 1,1 eq) est additionné. Au bout d'une nuit d'agitation la réaction est terminée, Les solvants sont évaporés au rotavapor. Le résidu d'évaporation est repris avec 100 ml d'éther éthylique pour être mis en suspension, puis essoré sur fritté, le gâteau est rincé avec un minimum d'éther. Note : en CCM on observe une petite trace de biotine mais ça n'aura pas d'impact pour la suite.
m = 7,106g
Rendement : 81%
Eluant CCM : DCM/MeOH : 90/10

### → Couplage entre la biotine et l'amine aromatique (composé 16)

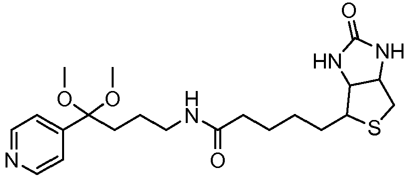

Le composé 15 (808 mg ; 2,0 mmol ; 1,4 eq) est solubilisé dans le DMF (4,28 ml) à 60°C, avant d'ajouter de la triéthylamine (1,60 ml ; 11,4 mmol ; 8,0 eq), après 5 minutes d'agitation. Le composé 20 en solution dans le DMF (2,85 ml à 500 mM, soit 300 mg ; 1,4 mmol ; 1,0 eq) est incorporé en une fois. Après 60 minutes, le solvant est évaporé sous pression réduite. On ne traite pas d'avantage, le brut réactionnel est directement engagé en déprotection.

La masse et le rendement seront calculés à l'étape suivante.
Méthode HPLC : neutre

### → Cétone biotinylée (composé 17)

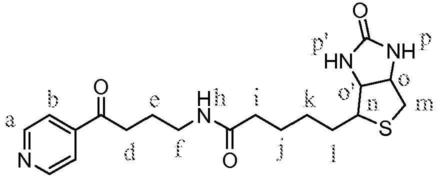

Le composé **16** est repris dans 50 ml d'une solution 6 M d'acide chlorhydrique agité pendant 2 heures à température ambiante. La solution est évaporée à sec, le résidu est une huile rouge sous forme de chlorhydrate. Il est repris dans un mélange DCM/MeOH/TEA : 90/8/2, l'huile se décolore et un gaz se dégage, 2 ml de triéthylamine sont rajoutés puis le mélange est évaporé à sec pour donner un précipité qui est repris dans 100 ml d'éther éthylique et 10 ml de DCM, la suspension est essorée et le gâteau est trituré avec 50 ml d'éther avant d'être mis à l'étuve sous vide 2 heures à 28°C. On reprend la poudre dans 5 ml de mélange DCM/MeOH : 88/12 auxquels 250 µl de soude 2 M sont additionnés afin de solubiliser le tout. Cette solution est déposée sur une colonne de gel de silice Si60 de diamètre = 3 cm, h = 16 cm v = 5 cm/minute et qui est éluée avec un mélange DCM/MeOH : 88/12.
m = 940 mg
Rendement : 83% sur deux étapes, et si on ne prend pas en compte les 2,9 eq de chlorhydrate de triéthylamine.
Eluant CCM : DCM/MeOH : 85/15.
Méthode HPLC : neutre
**RMN ¹H** (200 MHz, DMSO) : δ = 1,28 (t ; TEA) ; 1,49 (M; 2H ; k) ; 1,30 à 1,50 (m; 4H ; j et l) ; 1,73 (quint; 2H ; e) ; 2,05 (t; 2H ; i) ; 2,6 à 2,8 (M; 2H ; m) ; 2,9 à 3,2 (m ; 5H + H du TEA ; d, f, n et TEA) ; 4,0 à 4,3 (m ; 2H ; o et o') ; 6,38 (s ; 1H ; p') ; 6,43 (s ; 1H ; p) ; 7,78 (d ; 2H ; b) ; 7,95 (t; 1H ; h) ; 8,08 (d ; 2H ; a) ; 10,3 (M ; le chlorhydrate du TEA) .

### → Hydrazonation de la cétone (18)

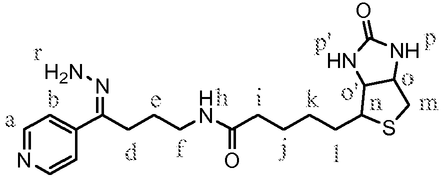

La cétone 17 (400 mg ; 506,5 µmol) est dissoute dans du DMF (2,25 ml ; 224 mM), du MeOH (11,3 ml ; 44,8 mM) et de l'acide acétique (580 µl ; 10,13 mmol ; 20 eq) avant d'ajouter 247 µl d'hydrazine monohydrate (246 µL ; 5,065 mmol ; 10 eq). La solution est agitée pendant deux heures puis évaporée à sec.

Le résidu d'évaporation est repris dans 5 ml d'eau puis transvasé dans un flacon de 15 ml pour ensuite être basifié à la soude 2 M jusqu'à atteindre un pH de 10, le produit précipite, le tube est agité par vortex, afin de bien mélanger puis centrifugé à 8000 tr/min. Le surnageant est jeté puis l'opération est répété encore deux fois en prenant 5 ml d'une solution de soude à pH 10. Le précipité (poudre jaune) est séché dans une étuve sous vide à température ambiante avant d'être engagé dans l'étape d'oxydation.
m = 190 mg
Rendement : 93%
Méthode HPLC : neutre puis basique.
**RMN ¹H** (200 MHz, DMSO) : δ = 1,2 à 1,7 (m; 8H ; e, j, k et l) ; ; 1,73 (quint; 2H ; e) ; 2,06 (t; 2H ; i) ; 2,6 à 2,9 (M; 2H ; m) ; 3,0 à 3,2 (m ; 3H ; f et n) ; 4,10 à 4,35 (m ;2H ; o et o') ; 6,39 (s ; 1H ; p') ; 6,47 (s ; 1H ; p) ; 7,11 (s ; 2H ; r) ; 7,55 (d ; 2H ; b) ; 7,87 (t; 1H ; h) ; 8,47 (d ; 2H ; b) .

### → Pyridine Diazo Cetone Biotinylée (PyDCB) (19)

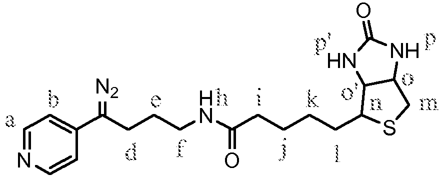

L'hydrazone 18 (177,2 mg ; 438 µmol ; 1,0 eq) est dissoute partiellement dans du DMF (22 ml ; 20 mM) avant d'être refroidie à 0°C pour que le tétraméthylguanidine (448 µl ; 3,56 mmol ; 8,14 eq), le tamis moléculaire 3Â (722 mg ; 3,8 fois la masse du réactif mis en jeu) et l'oxyde de manganèse (2,387 g ; 35,6 mmol ; 81,4 eq) soient ajoutés. La suspension est agitée 20 minutes, puis filtrée sur un bouchon de célite d'1 cm de haut, le bouchon est rincé au méthanol jusqu'à ce que le filtrat coule incolore. La solution est évaporée à sec puis reprise avec 20 ml de DCM/MeOH : 90/10 et lavé avec une solution de Na₂CO₃ à 0,25 M. Un précipité se forme. Après analyse, le précipité et la phase organique sont assemblés et évaporés à sec. Afin d'être dissout dans 100 ml de mélange de DCM/MeOH : 90/10 et lavé avec 100 ml de Na₂CO₃ à 0,05 M. La phase organique est récupérée, séchée au Na₂CO₃ anhydre, filtrée et évaporée à sec.
m = 42,8 mg
Rendement : 24,3%
Méthode HPLC : basique.
**RMN ¹H** (200 MHz, DMSO) : δ = 1,20 à 1,74 (m; 8H ; e, j, k et l) ; ; 1,73 (quint; 2H ; e) ; 2,07 (t; 2H ; i) ; 2,6 à 2,9 (M; 2H ; m) ; 3,00 à 3,25 (m ; 3H ; f et n) ; 4,10 à 4,35 (m ;2H ; o et o') ; 6,37 (s ; 1H ; p') ; 6,44 (s ; 1H ; p) ; 6,94 (d ; 2H ; b) ; 7,90 (t; 1H ; h) ; 8,35 (d ; 2H ; b).

### Résultats et conclusions :

**Nous avons démontré que la synthèse d'un composé comportant une fonction diazo méthyle conjuguée à un noyau pyridine en *alpha* et reliée à un groupement de reconnaissance (biotine) en *alpha'* est réalisable avec un bon rendement.**

### Exemple 3 : Démonstration de la stabilité du PyDB ou du PyDCB par rapport à une molécule de BBP (bis-bio-PDAM) en milieu liquide à température ambiante :

### Objectif :

L'objectif est de démontrer la stabilité en milieu liquide des molécules PyDB ou PyDCB en comparaison à une molécule de deuxième génération. Pour cela une étude de stabilité accélérée est effectuée dans des conditions extrêmes où les composés sont conservés à 125 mM dans un mélange DMSO/méthanol 96/4 à température ambiante (22°C +/- 1°C). A noter que ce sont des conditions extrêmes de conservation.

### Mode Opératoire :

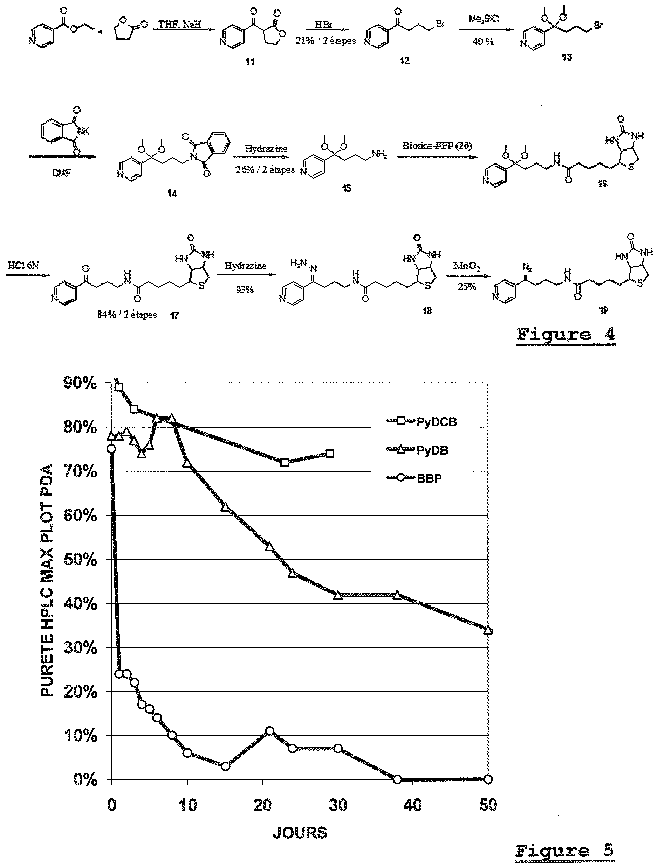

Les trois composés PyDB, PyDCB et BBP, représentés sur la Figure 2, sont solubilisés à 125 mM dans un mélange DMSO/méthanol 96/4 et conservés à température ambiante (22°C à plus ou moins 1°C). On injecte alors régulièrement en HPLC (méthode basique, chaîne HPLC Waters) 2 µL de ces solutions, afin de mesurer la dégradation du produit principal par intégration de l'ensemble des pics du chromatogramme (PDA Max Plot sur le logiciel Empower 2). On reporte alors l'évolution de la pureté du composé initial en fonction du temps, comme cela est bien représenté sur la Figure 5.

### Résultats et conclusions :

**On démontre sans ambiguïté que le BBP se dégrade en quelques jours (< 10% de pureté à 10 jours) alors que les deux molécules de quatrième génération PyDB et PyDCB sont encore stables à plus de 80% au bout de ce laps de temps.**

**La présence du motif pyridinyle en *alpha* de la fonction diazo stabilise cette dernière, par une délocalisation électronique qui rend la fonction diazo moins sensible à l'hydrolyse. La présence du marqueur en *alpha'* stabilise encore considérablement cette fonction en la rendant moins susceptible à l'hydrolyse en milieu aqueux.**

### Exemple 4 : Démonstration de la stabilité du PyDB par rapport à une molécule de BBP, sous forme sèche à +4°C :

### Objectif :

Démontrer la stabilité à sec des molécules PyDB ou PyDCB en comparaison à une molécule BBP de deuxième génération.

### Mode Opératoire :

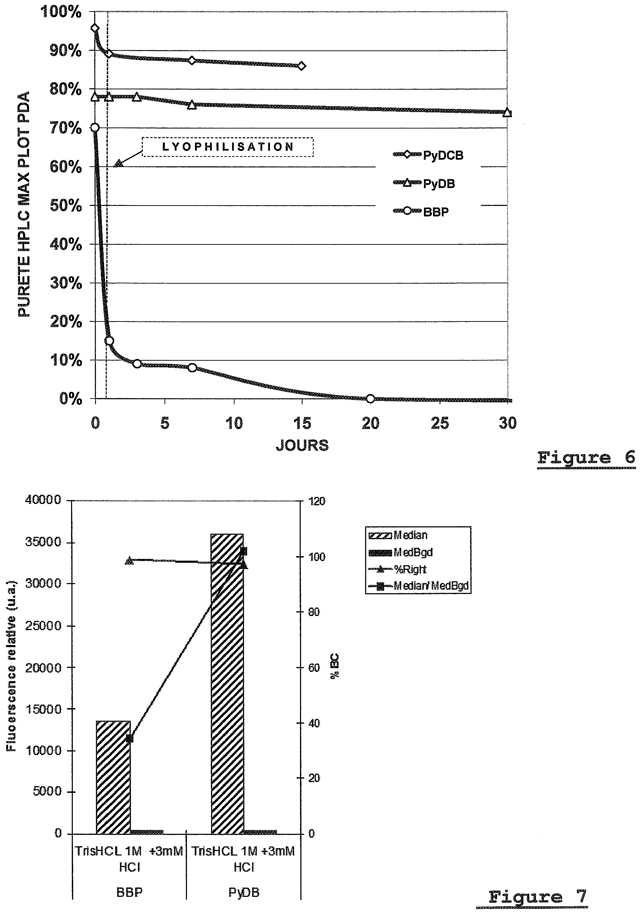

Les deux composés PyDB et le BBP sont solubilisés à 250 mM dans une solution de Tris HCl 10 mM pH 7,5 et de Tréhalose à 10 %. Les solutions sont lyophilisées pendant une nuit par aliquots de 50 nmoles. Les produits secs sont ensuite conservés à +4°C. Régulièrement, on solubilise ces aliquots dans du méthanol et on injecte en HPLC (Waters) 15 µL de ces solutions, afin de mesurer la dégradation du produit principal par intégration de l'ensemble des pics du chromatogramme (PDA Max Plot sur le logiciel Empower). On reporte alors l'évolution de la pureté du composé initial en fonction du temps, comme cela est bien représenté sur la Figure 6.

### Résultats et conclusions :

**De la même manière que dans l'exemple 3, mais de façon encore plus notable, on démontre que le BBP ne supporte pas la lyophilisation (> 60% de dégradation pendant cette étape) alors que le PyDB et le PyDCB restent parfaitement stables pendant cette opération. De plus, un séjour de plus d'un mois à l'état sec et à 4°C ne montre que très peu de dégradation (supérieure à 80% de pureté pour les deux marqueurs de quatrième génération).**

**Encore une fois on démontre que la présence du motif pyridinyle en *alpha* de la fonction diazo stabilise cette dernière, par une délocalisation électronique qui rend la fonction diazo moins sensible à l'hydrolyse.**

### Exemple 5 : Marquage d'acides nucléiques par du PyDB par rapport à une molécule de BBP avec purification intermédiaire :

### Objectif :

Démontrer l'efficacité du marquage des acides nucléiques par une molécule PyDB en comparaison à une molécule de deuxième génération (BBP).

Pour cela des amplicons d'ARN, fragments de la séquence de *Mycobacterium tuberculosis* de 174 bases, issus d'une réaction d'amplification (NASBA, kit NucliSens Basic Kit de bioMérieux B.V.- Boxtel - Pays-Bas) sont marqués à la biotine par réaction avec des marqueurs diazo. Les produits de la réaction de marquage sont détectés par hybridation sur une puce à ADN d'Affymetrix (Custom DNA Chip Combo Myco décrite dans J. Clin. Microbiol., 37(1), PP49-55, A. Troesch et al., 1999).

### Mode Opératoire :

Dans un tube, on mélange :
- 18 µL de solution de marqueur à 250 mM (DMSO/méthanol 96/4), il s'agit soit du PyDB soit du BBP,
- 12 µL de DMSO,
- 15 µL de tampon NASBA 0,5X (Kit "NucliSens Basic Kit Easy Q bioMérieux"),
- 35 µL de tris HCL 1 M,
- 5 µL de NASBA 0,1 X (réaction d'amplification NASBA diluée dix fois, amplicon de 174 bases), et
- 15 µL de HCl 20 mM.

La solution est mélangée parvortex puis incubée 10 minutes à 65°C.

### Purification des acides nucléiques :

Les acides nucléiques marqués ont été purifiés sur colonne QiaQuick (PCR purification kit QiaQuick, Qiagen, Hilden, Allemagne) en utilisant le protocole de purification recommandé par le fabricant. Le volume d'élution est de 100 µL.

### Hybridation sur puce à ADN :

Après purification, les acides nucléiques marqués sont transférés dans 400 µL de tampon d'hybridation. Les échantillons sont hybridés sur les puces à ADN conçues pour l'analyse de la séquence M20940 "GenBank" de l'ARN 16S de *Mycobacterium tuberculosis.* Cette puce à ADN est décrite par A. Troesch et al. dans un article publié dans J. Clin. Microbiol., 37(1), PP49-55, 1999.

Les étapes d'hybridation ont été réalisées sur les stations fluidiques FS 450 (Affymetrix, Santa Clara, CA) en utilisant le protocole d'hybridation et les tampons décrits dans la publication précédente d'A. Troesch *et al*.

L'hybridation est révélée par le couplage de la streptavidine (SA) marquée à la phycoérythrine (PE), qui interagit avec la biotine des marqueurs utilisés dans les conditions suivantes : 300 µL d'eau pure ; 300 µL de tampon tris 100 mM pH 7 / NaCl 1 M/tween 20 0,05% / antimousse 0,005% ; 6 µL de BSA (50 mg/mL) ; 6 µL de SA-PE (300 µg/mL).

### Lecture de la puce à ADN :

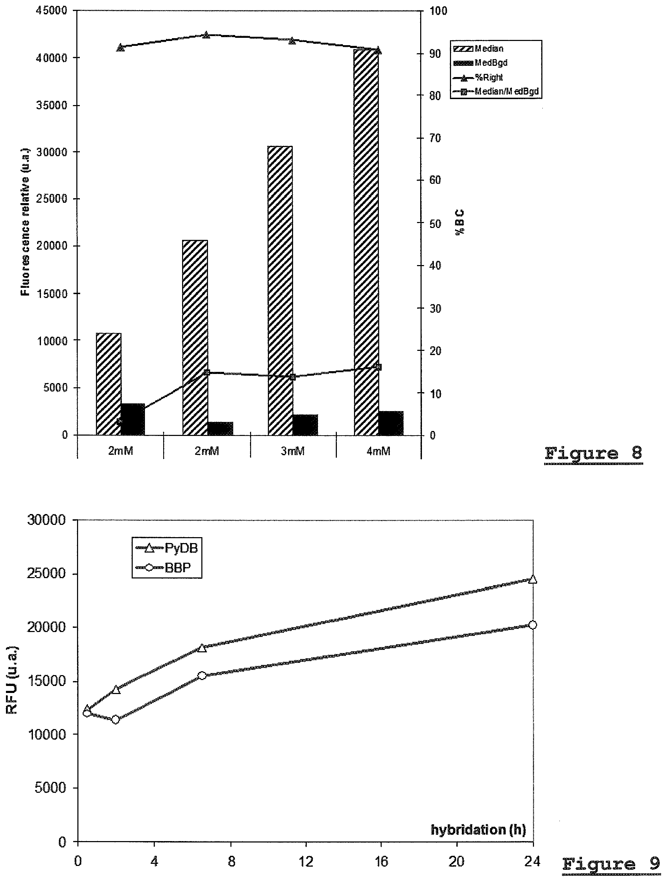

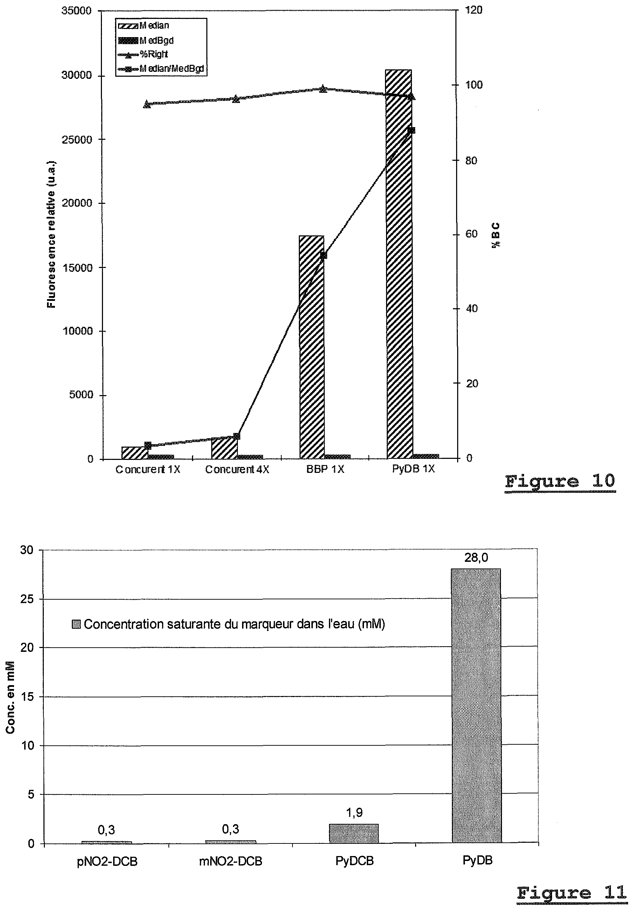

La lecture de la fluorescence émise à la surface de la puce à ADN après marquage et hybridation ainsi que la génération des données en termes d'intensité du signal et de pourcentage d'homologie sont réalisés par les systèmes de lecture et le logiciel fournis par Affymetrix (Scanner Gene Chip Array et Logiciel GCOS). Le système de lecture fournit des intensités en signal et bruit de fond exprimées en rfu (« relative fluorescence unit », ou « unité de fluorescence relative »). Le pourcentage d'homologie (%BC ou %Right sur la Figure 7 mais également ultérieurement sur les Figures 8 et 10) est donné par rapport à une séquence de référence qui est dans ce cas la séquence de *Mycobacterium tuberculosis.*

Les résultats en termes d'intensité médiane du signal (Med), du bruit de fond (Med Bckgd) et du pourcentage d'homologie (%Right) sont donnés dans le graphique de la Figure 7 pour les marqueurs BBP et PyDB.

D'une manière générale, c'est un pourcentage d'homologie supérieur à 90% qui est recherché en priorité. En second lieu, c'est un signal spécifique élevé et un bruit de fond faible qui sont souhaités.

### Résultats et conclusions :

**Cet exemple montre que le marqueur PyDB donne un signal fluorescent trois fois supérieur au signal donné par la molécule BBP de référence.**

**Compte tenu de la très grande stabilité du PyDB, ce marqueur démontre sa supériorité par rapport aux molécules des précédentes générations.**

### Exemple 6 : Marquage d'acides nucléiques par le PyDB par rapport à une molécule de BBP, sans purification intermédiaire :

### Objectif :

On cherche à démontrer la même chose que dans l'exemple 5, mais en se plaçant dans des conditions défavorables, où l'excès de marqueur n'est pas éliminé par purification.

### Mode Opératoire :

Dans un tube de 1 mL, on mélange :
- 5 µL de solution de marqueur à 8 mM (DMSO/Méthanol 96/4). Il s'agit soit du PyDB soit du BBP,
- 5 µL de NASBA 0.1 X (Kit NucliSens Basic Kit de bioMérieux),
- 5 µL de tris HCL 1M, et
- 5 µL de HCl 20 mM.

La solution a été mélangée par vortex puis incubée 10 minutes à 65°C. Les acides nucléiques ainsi marqués ne sont pas purifiés, mais directement hybridés.

### Hybridation sur puce à ADN :

Les acides nucléiques marqués sont transférés sans purification dans 480 µL de tampon d'hybridation. Les échantillons sont hybridés sur les puces à ADN de la même façon que pour l'exemple précédent.

### Lecture de la puce à ADN :

Les résultats en termes d'intensité, de bruit de fond et de pourcentage d'homologie, pour les marqueurs BBP et PyDB, sont présentés dans le graphique de la Figure 8.

### Résultats et conclusions :

**Cet exemple montre que le marqueur PyDB présente, du fait de sa structure, un bruit de fond amoindri par rapport à la molécule de référence BBP. Les signaux spécifiques significatifs d'une hybridation spécifique sont également améliorés et le pourcentage d'homologie, relativement faible pour le BBP, devient plus important pour le PyDB (à 2 mM).**

**Une fois encore, la supériorité des molécules de quatrième génération par rapport à celles des précédentes générations est démontrée.**

### Exemple 7 : Evaluation de la stabilité sur 24 heures d'un amplicon marqué par le PyDB :

### Objectif :

On cherche à démontrer qu'un amplicon ARN marqué, peut être hybridé jusqu'à 24 heures sur une puce à ADN sans perdre de son intensité de fluorescence. Ce qui démontre la stabilité de la liaison ARN-marqueur.

### Mode Opératoire :

Dans un tube, on mélange :
- 5 µL de solution de marqueur (pyDB ou BBP) à 10 mM dans le DMSO/MeOH (96/4),
- 5 µL de produit d'amplification NASBA 0,1 X (Kit NucliSens Basic Kit de bioMérieux),
- 5 µL de Tris HCL 1 M, et
- 5 µL d'eau.

La solution est mélangée par vortex puis incubée 10 minutes à 65°C.

### Purification des acides nucléiques :

Les acides nucléiques marqués ont été purifiés sur colonne QiaQuick (PCR purification kit, Qiagen) en utilisant le protocole de purification recommandé par le fabricant. Le volume d'élution est de 100 µL.

### Hybridation sur puce à ADN :

Après purification, les acides nucléiques marqués sont transférés dans 400 µL de tampon d'hybridation. Les échantillons sont hybridés sur les puces à ADN conçues pour l'analyse de la séquence M20940 "GenBank" de l'ARN 16S de *Mycobacterium tuberculosis.* Cette puce à ADN est décrite dans la publication d'A. Troesch et al., J. Clin. Microbiol., 37(1), PP49-55, 1999.

Les étapes d'hybridation ont été réalisées en injectant 80 µL du mélange d'hybridation dans la puce puis en maintenant celle-ci dans un four à hybrider à 45°C pendant 0,5 heure, 2 heures, 6,5 heures ou 24 heures.

L'hybridation est révélée par le couplage de la streptavidine (SA) marquée à la phycoérythrine (PE), qui interagit avec la biotine des marqueurs utilisés dans les conditions suivantes : 300 µL d'eau pure ; 300 µL de tampon tris 100 mM pH 7 / NaCl 1 M / tween 20 0,05% / antimousse 0,005% ; 6 µL de BSA (50 mg/mL) ; 6 µL de SA-PE (300 µg/mL).

### Lecture de la puce à ADN :

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix. Le système de lecture fournit des intensités en signal et bruit de fond exprimées en rfu ("relative fluorescence unit", ou "unité de fluorescence relative"). Le pourcentage d'homologie est donné par rapport à une séquence de référence qui est dans ce cas la séquence de *Mycobacterium tuberculosis.*

Les résultats en termes d'intensité médiane du signal (Med), en fonction de la durée d'hybridation sont donnés sur la Figure 9 pour le marqueur de quatrième génération, PyDB.

Ils montrent que le signal de fluorescence reste stable, voire à tendance à monter en fonction de la durée de l'hybridation.

### Résultats et conclusions :

**Cet exemple montre que les amplicons marqués avec le PyDB restent parfaitement stable au cours de l'hybridation qui peut être prolongée pendant 24H (intérêt particulier pour les hybridations longues et expression des gènes en cancérologie), voir la** **Figure 9****.**

**On observe même une augmentation du signal de fluorescence au cours du temps qui est due à une meilleure hybridation des amplicons (la cinétique d'hybridation est lente).**

**On démontre ainsi la stabilité de la liaison marqueur-acide nucléique.**

### Exemple 8 : Comparaison de l'efficacité de marquage des molécules décrites dans la présente invention par rapport à celle d'une technologie commercialement disponible (ULS RNA Labelling Kit):

### Mode Opératoire :

Les amplicons ARN sont préparés par une amplification NASBA comme précédemment et sont marqués avec les molécules BBP et PyDB.

Dans un tube, on mélange :
- 5 µL de NASBA 1X (Kit NucliSens Basic Kit de bioMérieux),
- 5 µL de solution de marqueur à 20 mM (BBP, ou PyDB dans le DMSO/Méthanol 96/4,
- 5 µL de Tris HCL 1 M pH 7,4 et
- 5 µL d'eau.

La solution a été mélangée par vortex puis incubée 10 minutes à 65°C.

Pour le marquage avec le kit commercial « ULS RNA Labelling Kit » de Kreatech (Amsterdam, Pays-Bas), le protocole recommandé par le fournisseur a été suivi. En résumé, on mélange :
- 20 µL de NASBA 1X (Kit NucliSens Basic Kit, bioMérieux B.V., Boxtel, Pays-Bas),
- 1 µL de solution de marqueur,
- 3 µL Tampon 10x, et
- 6 µL d'eau.

La solution a été incubée 30 minutes à 85°C

### Purification des acides nucléiques :

Les acides nucléiques marqués à l'aide des molécules BBP ou PyDB ont été purifiés sur colonne QiaQuick (PCR purification kit, Qiagen) en utilisant le protocole de purification recommandé par le fabricant. Le volume d'élution est de 100 µL. Pour les acides nucléiques marqués à l'aide du kit commercial, ULS RNA Labelling Kit, la purification recommandée et fournie par Kreatech (Amsterdam, Pays-Bas) a été utilisée. Le volume final est de 30 µL, auxquels on ajoute 100 µL d'une solution de blocage recommandée par cette société.

### Hybridation sur puce à ADN :

Après purification, les acides nucléiques marqués sont transférés dans 400 µL de tampon d'hybridation (BBP ou PyDB) ou 370 µL de tampon d'hybridation de Kreatech.. Lesdits acides nucléiques sont hybridés sur les puces à ADN conçues pour l'analyse de la séquence M20940 « GenBank » de l'ARN 16S de *Mycobacterium tuberculosis.*

Cette puce à ADN est décrite dans la publication de A. Troesch et al., J. Clin. Microbiol., 37(1), PP49-55, 1999. Les étapes d'hybridation ont été réalisées sur les stations fluidiques (Affymetrix FS 450) en utilisant le protocole d'hybridation et les tampons décrits dans cette publication de A. Troesch *et al*. L'hybridation est révélée par le couplage de la streptavidine (SA) marquée à la phycoérythrine (PE), qui interagit avec la biotine des marqueurs utilisés dans les conditions suivantes : 300 µL d'eau pure ; 300 µL de tampon tris 100 mM pH 7 / NaCl 1 M / tween 20 à 0,05% / antimousse 0,005% ; 6 µL de BSA (50 mg/mL) ; 6 µL de SA-PE (300 µg/mL).

### Lecture de la puce à ADN :

La lecture de la fluorecence émise à la surface de la puce à ADN après marquage et hybridation ainsi que la génération des données en termes d'intensité du signal et de pourcentage d'homologie sont réalisés par les systèmes de lecture et le logiciel fourni par Affymetrix (Gen Chip array et logiciel GCOS). Le système de lecture fournit des valeurs médianes des intensités en signal (Median) et bruit de fond (MedBgd) exprimées en rfu ("relative fluorescence unit", ou "unité de fluorescence relative"). Le pourcentage d'homologie (% right), appelé aussi « base call percentage » (%BC) est donné par rapport à une séquence de référence qui est dans ce cas la séquence de *Mycobacterium tuberculosis.*

Les résultats en termes d'intensité médiane du signal (Med), du bruit de fond (MedBgd) et du pourcentage d'homologie (%Right ou %BC) sont donnés dans la Figure 10 pour les marqueurs BBP, PyDB ainsi que pour le kit concurrent.

### Résultats et conclusions :

**On constate que la technologie utilisant les marqueurs cisplatine (ULS RNA Labelling Kit), appliquée exactement dans les conditions décrites par le fournisseur, à un potentiel de marquage bien inférieur à la solution technique apportée par la présente invention, puisqu'avec ce kit commercial il faut rajouter plus de quatre fois la concentration d'ARN pour avoir un signal nettement détaché du bruit de fond (Concurrent 4X,** **Figure 10****), mais qui reste, dans tous les cas plus, dix fois plus faible que le marquage réalisé avec les molécules BBP et dix-huit fois plus faible que le marquage réalisé avec les molécules PyDB.**

**Dans tous les cas le pourcentage d'identification (%BC ou %Right) reste le même.**

**Le marquage sur les liens internucléosidiques en comparaison à une autre technique de marquage permet donc d'obtenir une sensibilité de détection bien meilleure et cela quelles que soient les générations de molécules. La quatrième génération montre, quant à elle, une nouvelle fois sa supériorité dans ce nouvel exemple.**

### Exemple 9 : Comparaison de la solubilité de deux marqueurs diazo de troisième génération (para Nitro DCB et meta Nitro DCB) et de deux marqueurs de quatrième génération (PyDB et PyDCB) :

### Objectif :

On veut démontrer que la présence du noyau pyridine sur un réactif de marquage diazo améliore la solubilité de la molécule.

### Mode Opératoire :

### Détermination des Epsilons (ε) molaires des marqueurs :

On pèse précisément quelques milligrammes de marqueur (5-15 mg) que l'on solubilise à 100 mM dans le DMSO (entièrement soluble). On dilue au 1/10^{ème} cette solution dans le MeOH puis à nouveau au 1/100^{ème} dans le MeOH (au final dilution au 1/1000^{ème}). On mesure le spectre UV de cette solution, afin de déterminer le λₘₐₓ, auquel on mesure l'absorbance. Cette mesure permet de calculer l'ε molaire de chaque marqueur (loi de Beer Lambert) .

### Détermination de la solubilité dans l'eau des marqueurs :

On pèse précisément quelques mg de marqueur (5-15 mg) que l'on solubilise à 100 mM dans de l'eau (partiellement soluble). On agite à l'aide d'un vortex et on centrifuge. On prélève le surnageant que l'on dilue au 1/10^{ème} ou au 1/100^{ème} dans le MeOH. On mesure le spectre UV des marqueurs en s'assurant qu'il n'y a pas eu de dégradation. On mesure l'absorbance au λₘₐₓ et on calcule la concentration du marqueur dans le surnageant en utilisant la valeur de l'ε molaire déterminée précédemment, soit la solubilité du marqueur dans l'eau (Figure 11)

### Résultats et conclusions :

**On voit sur la** **Figure 11** **que le PyDB est six fois plus soluble dans l'eau que les molécules de troisième génération *para* Nitro DCB et *meta* Nitro DCB. Ce gain de solubilité est uniquement dû à la présence du noyau pyridinyle. Le PyDB est quatre-vingt-dix fois plus soluble que les molécules de troisième génération *para* Nitro DCB et *meta* Nitro DCB. Ce gain de solubilité est également apporté en majeure partie par le noyau pyridine bisubstitué.**

**Nous avons ainsi démontré l'impact très important du noyau pyridinyle sur la solubilité finale de ces molécules.**

Il s'avère que le noyau pyridine est un noyau connu pour sa basicité, on pouvait prévoir une stabilisation de la fonction diazométhyle mais *a contrario* un manque de réactivité vis-à-vis des acides nucléiques. En effet le mécanisme de la réaction diazo/phosphate est basé sur un échange de proton. La présence d'un noyau piégeur de proton (pyridine) aurait pu inhiber la réaction, or il a été observé que ce phénomène n'apparaissait qu'avec des bases plus fortes comme la pipéridine. La réaction de marquage s'effectue à pH 7 en milieu tamponné.

Dans l'exemple ou le marqueur est en *alpha*', on pouvait s'attendre à des problèmes de réactivité avec les phosphates en raison de problèmes de gène stérique accrus. Là encore l'effet de ces molécules est surprenant.

De plus la génération de diastéréoisomères lors de l'alkylation des phosphates risquait de perturber l'hybridation et donc la détection des acides nucléiques. Ceci était prévisible et laissait penser que ce type de marqueur serait moins efficace. Il était donc non évident de réaliser ce type de molécules et d'obtenir d'aussi bons résultats de marquage.

## Revendications

1. Réactif de marquage de formule (C) : 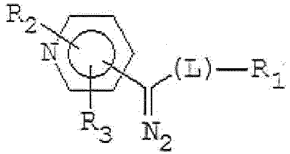 dans laquelle :
• R₁ représente un marqueur détectable capable de générer directement ou indirectement un signal détectable et sélectionné parmi :
- les enzymes qui produisent un signal par colorimétrie, fluorescence ou luminescence,
- les chromophores,
- les groupements à densité électronique détectable par microscopie électronique ou par leur propriété électrique,
- les groupements détectables électro-chimiquement,
- les dérivés d'un complexe de fer,
- les molécules radioactives,
- les couples ligand/anti-ligands choisis parmi les couples suivants : biotine/streptavidine, haptène/anticorps, antigène/anticorps, peptide/anticorps, sucre/lectine, polynucléotide/complémentaire du nucléotide,
- les composés fluorescents de poids moléculaire inférieur à 1000g/mole,
- les haptènes de poids moléculaire inférieur à 1000g/mole,
- les fluorophores,
• R₂ et R₃ représentent indépendamment l'un de l'autre : H, CONHR, COOR avec R correspondant à un groupe hydrocarboné aliphatique saturé comprenant de un à six atomes de carbone ou à un groupe aromatique monocyclique, et
• L est un bras de liaison comportant un enchaînement linéaire d'au moins deux liaisons covalentes comprenant un motif -(O-CH₂-CH₂)-, répété de 1 à 20 fois.

2. Réactif de marquage de formule (D) : 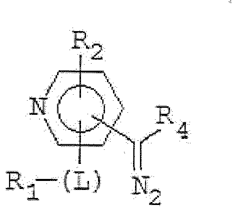 dans laquelle :
• R₁ représente un marqueur détectable capable de générer directement ou indirectement un signal détectable et sélectionné parmi :
- les enzymes qui produisent un signal par colorimétrie, fluorescence ou luminescence,
- les chromophores,
- les groupements à densité électronique détectable par microscopie électronique ou par leur propriété électrique,
- les groupements détectables électro-chimiquement,
- les dérivés d'un complexe de fer,
- les molécules radioactives,
- les couples ligand/anti-ligands choisis parmi les couples suivants : biotine/streptavidine, haptène/anticorps, antigène/anticorps, peptide/anticorps, sucre/lectine, polynucléotide/complémentaire du nucléotide,
- les composés fluorescents de poids moléculaire inférieur à 1000g/mole,
- les haptènes de poids moléculaire inférieur à 1000g/mole,
- les fluorophores,
• R₂ représente : H, CONHR, COOR avec R correspondant à un groupe hydrocarboné aliphatique saturé comprenant de un à six atomes de carbone ou à un groupe aromatique monocyclique,
• R₄ représente : H, un groupe hydrocarboné aliphatique saturé comprenant de un à six atomes de carbone ou un groupe aromatique monocyclique, et
• L est un bras de liaison comportant un enchaînement linéaire d'au moins deux liaisons covalentes comprenant un motif -(O-CH₂-CH₂)-, répété de 1 à 20 fois.

3. Réactif de marquage de formule (E) : 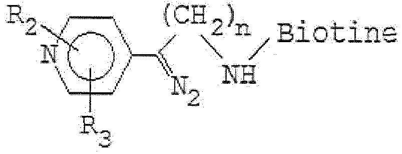 dans laquelle :
• R₂ et R₃ représentent indépendamment l'un de l'autre : H, CONHR, COOR avec R correspondant à un groupe hydrocarboné aliphatique saturé comprenant de un à six atomes de carbone ou à un groupe aromatique monocyclique, et
• n est un nombre entier compris entre 1 et 12.

4. Réactif de marquage de formule (F) : 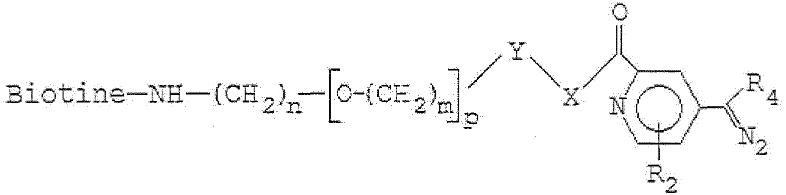 dans laquelle :
• R₂ représente : H, CONHR, COOR avec R correspondant à un groupe hydrocarboné aliphatique saturé comprenant de un à six atomes de carbone ou à un groupe aromatique monocyclique,
• R₄ représente : H, un groupe hydrocarboné aliphatique saturé comprenant de un à six atomes de carbone ou un groupe aromatique monocyclique,
• -Y-X représente -CH₂NH-, -CONH-, -NHCO-, -CH₂O- ou -CH₂S-, et
• m, n et p sont chacun, indépendamment les uns des autres, un nombre entier compris entre 1 et 12.

5. Réactif de marquage selon la revendication 3, de formule (G) :

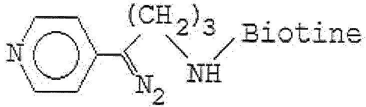

6. Réactif de marquage, selon la revendication 4, de formule (H) :

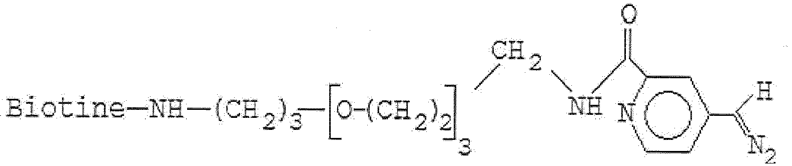

7. Réactif, selon l'une quelconque des revendications 1 à 2, **caractérisé par le fait que** R₁ est constitué par un résidu D-Biotine de formule (I) :

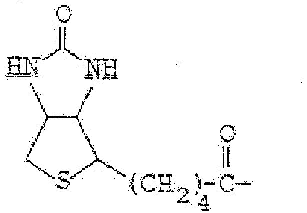

8. Procédé de synthèse d'un réactif de marquage, selon l'une quelconque des revendications 1, 3 ou 5, comprenant les étapes suivantes :
a) un dérivé d'acide carboxylique aromatique est mis en réaction avec l'énolate d'une lactone (réaction de type Claisen) pour former un précurseur cyclique,
b) lequel précurseur cyclique est ensuite ouvert avec un acide halogéné pour former une cétone aromatique halogénée,
c) la fonction carbonyle de la cétone aromatique halogénée est protégée par un groupement protecteur pour former un précurseur protégé,
d) lequel précurseur protégé est soumis à une réaction d'amination (de type Gabriel) pour former un précurseur aminé,
e) lequel précurseur aminé est déprotégé pour libérer la fonction amine, ladite fonction amine étant mise en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin
g) le précurseur marqué et carbonylé est transformé en un réactif de marquage, tel que décrit ci-dessus, par une transformation de la fonction carbonyle en une fonction diazo.

9. Procédé de synthèse d'un réactif de marquage, selon l'une quelconque des revendications 2, 4 ou 6, comprenant les étapes suivantes :
a) un acide aromatique di-carboxylique est estérifié pour former un diester,
b) ce diester est substitué régio-sélectivement avec un composé dérivé de l'éthylène glycol aminé mono protégé pour former un précurseur protégé sur la fonction carboxylique et sur la fonction amine,
c) lequel précurseur est ensuite réduit en alcool et oxydé en aldéhyde,
d) l'aldéhyde, synthétisé à l'étape c), est soumis à un traitement acide afin de, concomitamment protéger cette fonction tout en déprotégeant la fonction amine, pour conduire à un acétal aminé,
e) lequel acétal aminé est mis en réaction avec un marqueur détectable dont la fonction carboxylique est activée pour former un précurseur comportant un marqueur détectable,
f) le précurseur marqué est soumis à une réaction de déprotection de la fonction carbonyle pour former un précurseur marqué et carbonylé, enfin,
g) le précurseur marqué et carbonylé est transformé en un réactif de marquage, tel que décrit ci-dessus, par une transformation de la fonction carbonyle en une fonction diazo selon une réaction du type de Bamford Stevens.

10. Procédé pour le marquage d'une molécule biologique, comprenant la mise en contact en solution homogène, dans un tampon aqueux qui contient au moins 50% d'eau, d'une molécule biologique et d'un réactif, selon l'une quelconque des revendications 1 à 7.

11. Procédé de marquage et de fragmentation d'un acide nucléique simple ou double brin comprenant les étapes suivantes :
• fragmenter l'acide nucléique,
• attacher un marqueur sur au moins un des fragments par l'intermédiaire d'un réactif de marquage choisi parmi les réactifs, selon l'une quelconque des revendications 1 à 7, ledit réactif se couplant de manière covalente et majoritaire sur au moins un phosphate dudit fragment.

12. Procédé, selon la revendication 11, **caractérisé par le fait que** la fragmentation et le marquage sont effectués en deux étapes.

13. Procédé, selon la revendication 11, **caractérisé par le fait que** la fragmentation et le marquage sont effectués en une étape.

14. Procédé, selon l'une quelconque des revendications 11 à 13, **caractérisé par le fait que** le marquage s'effectue en solution homogène aqueuse qui contient au moins 50% d'eau.

15. Procédé, selon l'une quelconque des revendications 11 à 13, **caractérisé par le fait que** la fragmentation s'effectue par voie enzymatique, physique ou chimique.

16. Kit de détection d'un acide nucléique cible comprenant un acide nucléique marqué obtenu par le procédé tel que défini dans l'une quelconque des revendications 11 à 15.

17. Support solide sur lequel est fixé un réactif, selon l'une quelconque des revendications 1 à 7.

18. Procédé de capture d'acides nucléiques comprenant les étapes suivantes :
• on dispose d'un support solide sur lequel est fixé directement ou indirectement au moins une molécule biologique obtenue par le procédé tel que défini dans la revendication 10, ou un acide nucléique obtenu par le procédé tel que défini dans l'une quelconque des revendications 11 à 15, la molécule biologique ou l'acide nucléique comprenant une fonction diazométhyle,
• on met en contact un échantillon biologique susceptible de contenir des acides nucléiques libres, et
• on lave le support solide où la (ou les) molécule (s) sont fixée(s) de manière covalente au moins à un acide nucléique.

## Patentansprüche

1. Markierungsreagenz der Formel (C): 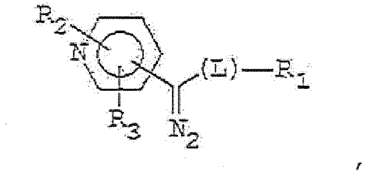 worin:
• R₁ eine nachweisbare Markierung darstellt, die in der Lage ist, direkt oder indirekt ein nachweisbares Signal zu erzeugen, und ausgewählt ist aus:
- Enzymen, die ein Signal mittels Kolorimetrie, Fluoreszenz oder Lumineszenz erzeugen,
- Chromophoren,
- elektronendichten Gruppen, die durch Elekronenmikroskopie oder durch ihre elektrische Eigenschaft nachweisbar sind,
- elektrochemisch nachweisbaren Gruppen,
- Derivaten eines Eisenkomplexes,
- radioaktiven Molekülen,
- Ligand/Antiligand-Paaren, die aus den folgenden Paaren ausgewählt sind: Biotin/Streptavidin, Hapten/Antikörper, Antigen/Antikörper, Peptid/Antikörper, Zucker/Lektin, Polynukleotid/Nukleotidkomplement,
- fluoreszierenden Verbindungen mit kleinerem Molekulargewicht als 1000 g/mol,
- Haptenen mit einem Molekulargewicht unter 1000 g/mol,
- Fluorophoren,
• R₂ und R₃ unabhängig voneinander Folgendes darstellen: H, CONHR, COOR, wobei R einer gesättigten aliphatischen Kohlenwasserstoffgruppe mit einem bis sechs Kohlenstoffatomen oder einer monozyklischen aromatischen Gruppe entspricht, und
• L ein Verbindungsarm ist, der eine gerade Abfolge von mindestens zwei kovalenten Bindungen enthält, die ein Motiv -(O-CH₂-CH₂)-, das 1 bis 20 Mal wiederholt ist, umfasst.

2. Markierungsreagenz der Formel (D): 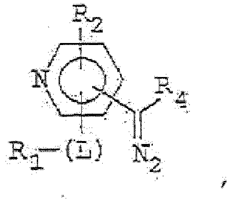 worin:
• R₁ eine nachweisbare Markierung darstellt, die in der Lage ist, direkt oder indirekt ein nachweisbares Signal zu erzeugen, und ausgewählt ist aus:
- Enzymen, die ein Signal mittels Kolorimetrie, Fluoreszenz oder Lumineszenz erzeugen,
- Chromophoren,
- elektronendichten Gruppen, die durch Elekronenmikroskopie oder durch ihre elektrische Eigenschaft nachweisbar sind,
- elektrochemisch nachweisbaren Gruppen,
- Derivaten eines Eisenkomplexes,
- radioaktiven Molekülen,
- Ligand/Antiligand-Paaren, die aus den folgenden Paaren ausgewählt sind: Biotin/Streptavidin, Hapten/Antikörper, Antigen/Antikörper, Peptid/Antikörper, Zucker/Lektin, Polynukleotid/Nukleotidkomplement,
- fluoreszierenden Verbindungen mit kleinerem Molekulargewicht als 1000 g/mol,
- Haptenen mit einem Molekulargewicht unter 1000 g/mol,
- Fluorophoren,
• R₂ Folgendes darstellt: H, CONHR, COOR, wobei R einer gesättigten aliphatischen Kohlenwasserstoffgruppe mit einem bis sechs Kohlenstoffatomen oder einer monozyklischen aromatischen Gruppe entspricht, und
• R₄ Folgendes darstellt: H, eine gesättigte aliphatische Kohlenwasserstoffgruppe mit einem bis sechs Kohlenstoffatomen oder eine monozyklische aromatische Gruppe, und
• L ein Verbindungsarm ist, der eine gerade Abfolge von mindestens zwei kovalenten Bindungen umfasst, die
• ein Motiv -(O-CH₂-CH₂)-, das 1 bis 20 Mal wiederholt ist, umfasst.

3. Markierungsreagenz der Formel (E): 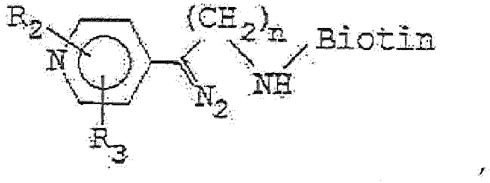 worin:
• R₂ und R₃ unabhängig voneinander Folgendes darstellen: H, CONHR, COOR, wobei R einer gesättigten aliphatischen Kohlenwasserstoffgruppe mit einem bis sechs Kohlenstoffatomen oder einer monozyklischen aromatischen Gruppe entspricht, und
• n eine ganze Zahl zwischen 1 und 12 ist.

4. Markierungsreagenz der Formel (F): 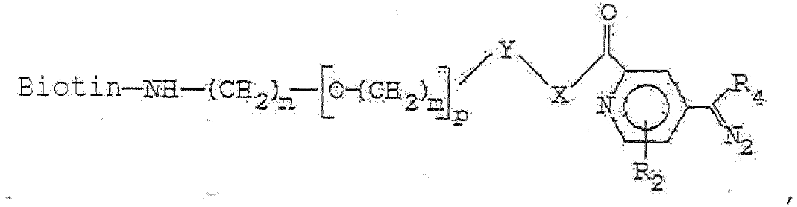 worin:
• R₂ Folgendes darstellt: H, CONHR, COOR, wobei R
• einer gesättigten aliphatischen Kohlenwasserstoffgruppe mit einem bis sechs Kohlenstoffatomen oder einer monozyklischen aromatischen Gruppe entspricht,
• R₄ Folgendes darstellt: H, eine gesättigte aliphatische Kohlenwasserstoffgruppe mit einem bis sechs Kohlenstoffatomen oder eine monozyklische aromatische Gruppe,
• -Y-X für -CH₂NH-, -CONH-, -NHCO-, -CH₂O- oder -CH₂S- steht und
• m, n und p jeweils unabhängig voneinander eine ganze Zahl zwischen 1 und 12 sind.

5. Markierungsreagenz nach Anspruch 3 der Formel (G):

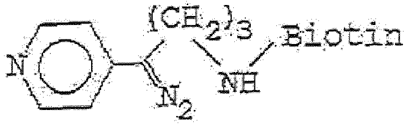

6. Markierungsreagenz nach Anspruch 4 der Formel (H):

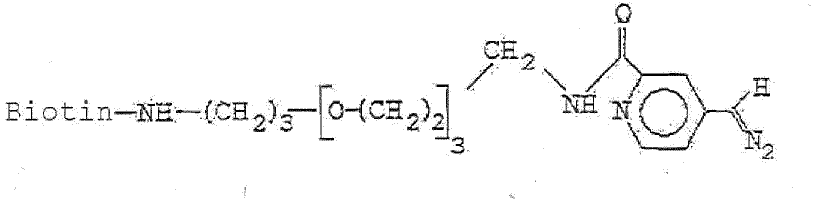

7. Reagenz nach einem der Ansprüche 1 bis 2, **dadurch gekennzeichnet, dass** R₁ aus einem D-Biotin-Rest der Formel (I) besteht:

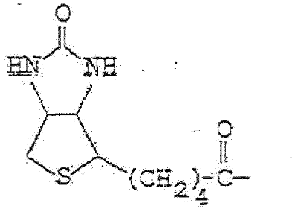

8. Verfahren zur Synthese eines Markierungsreagenzes nach einem der Ansprüche 1, 3 oder 5, das die folgenden Schritte umfasst:
a) ein aromatisches Carbonsäurederivat wird mit dem Enolat eines Lactons (Reaktion des Claisen-Typs) unter Herstellung eines zyklischen Vorläufers umgesetzt,
b) der zyklische Vorläufer wird anschließend mit einer Halogensäure unter Bildung eines aromatischen Halogenketons geöffnet,
c) die Carbonylfunktion des aromatischen Halogenketons wird durch eine Schutzgruppe unter Herstellung eines geschützten Vorläufers geschützt,
d) der geschützte Vorläufer wird einer Aminierungsreaktion (des Gabriel-Typs) zur Herstellung eines aminierten Vorläufers unterzogen,
e) von dem aminierten Vorläufer wird die Schutzgruppe abgespalten, um die Aminfunktion freizusetzen, wobei die Aminfunktion mit einer nachweisbaren Markierung umgesetzt wird, deren Carboxylfunktion aktiviert ist, so dass ein Vorläufer hergestellt wird, der eine nachweisbare Markierung trägt,
f) der markierte Vorläufer wird einer Reaktion zur Entfernung der Schutzgruppe von der Carbonylfunktion unterzogen, wobei ein markierter Vorläufer mit Carbonylgruppe hergestellt wird, und schließlich
g) wird der markierte Vorläufer mit Carbonylgruppe durch Umwandlung der Carbonylfunktion in eine Diazofunktion in ein Markierungsreagenz, wie vorstehend beschrieben, umgewandelt.

9. Verfahren zur Synthese eines Markierungsreagenzes nach einem der Ansprüche 2, 4, oder 6, das die folgenden Schritte umfasst:
a) eine aromatische Dicarbonsäure wird unter Herstellung eines Diesters verestert,
b) dieser Diester wird regioselektiv mit einem einfach geschützten aminierten Ethylenglykol-Derivat substituiert, wobei ein Vorläufer hergestellt wird, der an der Carboxylfunktion und an der Aminfunktion geschützt ist,
c) der Vorläufer wird dann zu dem Alkohol reduziert und zu dem Aldehyd oxidiert,
d) der in Schritt c) synthetisierte Aldehyd wird einer Säurebehandlung unterzogen, wobei diese Funktion geschützt und gleichzeitig die Schutzgruppe von der Aminfunktion entfernt wird, was zu einem aminierten Acetal führt;
e) das aminierte Acetal wird mit einer nachweisbaren Markierung umgesetzt, deren Carboxylfunktion aktiviert ist, so dass ein Vorläufer hergestellt wird, der eine nachweisbare Markierung enthält,
f) der markierte Vorläufer wird einer Reaktion zur Entfernung der Schutzgruppe von der Carbonylfunktion unter Bildung eines markierten Vorläufers mit Carbonylfunktion unterzogen und schließlich
g) wird der markierte Vorläufer mit Carbonylfunktion durch Umwandlung der Carbonylfunktion in eine Diazofunktion gemäß einer Reaktion des Bamford-Stevens-Typs in ein Markierungsreagenz, wie vorstehend beschrieben, umgewandelt.

10. Verfahren zur Markierung eines biologischen Moleküls, welches das Inkontaktbringen eines biologischen Moleküls und eines Reagenzes nach einem der Ansprüche 1 bis 7 in homogener Lösung in einem wässrigen Puffer, der mindestens 50% Wasser enthält, umfasst.

11. Verfahren zum Markieren und Fragmentieren einer einzel- oder doppelsträngigen Nukleinsäure, das die folgenden Schritte umfasst:
• Fragmentieren der Nukleinsäure,
• Anheften einer Markierung an mindestens eines der Fragmente mithilfe eines Markierungsreagenzes, das aus den Reagenzien nach einem der Ansprüche 1 bis 7 ausgewählt ist,
wobei das Reagenz kovalent und hauptsächlich an mindestens ein Phosphat des Fragments gebunden wird.

12. Verfahren nach Anspruch 11, **dadurch gekennzeichnet, dass** die Fragmentierung und die Markierung in zwei Schritten durchgeführt werden.

13. Verfahren nach Anspruch 11, **dadurch gekennzeichnet, dass** die Fragmentierung und die Markierung in einem Schritt durchgeführt werden.

14. Verfahren nach einem der Ansprüche 11 bis 13, **dadurch gekennzeichnet, dass** die Markierung in homogener wässriger Lösung, die mindestens 50% Wasser enthält, erfolgt.

15. Verfahren nach einem der Ansprüche 11 bis 13, **dadurch gekennzeichnet, dass** die Fragmentierung auf enzymatischem, physikalischem oder chemischem Weg erfolgt.

16. Kit zum Nachweis einer Zielnukleinsäure, umfassend eine durch das Verfahren nach einem der Ansprüche 11 bis 15 erhaltene markierte Nukleinsäure.

17. Fester Träger, auf dem ein Reagenz nach einem der Ansprüche 1 bis 7 gebunden ist.

18. Verfahren zum Einfangen von Nukleinsäuren, das die folgenden Schritte umfasst:
• man verfügt über einen fester Träger, auf dem direkt oder indirekt mindestens ein durch das Verfahren nach Anspruch 10 erhaltenes biologisches Molekül oder eine durch das Verfahren nach einem der Ansprüche 11 bis 15 erhaltene Nukleinsäure gebunden ist, wobei das biologische Molekül oder die Nukleinsäure eine Diazomethylfunktion umfasst,
• man bringt eine biologische Probe, die freie Nukleinsäuren enthalten kann, damit in Kontakt und
• man wäscht den festen Träger, auf dem das Molekül bzw. die Moleküle kovalent an mindestens eine Nukleinsäure gebunden ist/sind.

## Claims

1. Labelling reagent of formula (C): 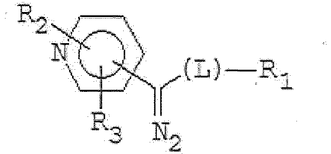 in which:
• R₁ represents a detectable label capable of directly or indirectly generating a detectable signal and selected among:
- enzymes which produce a detectable signal by colorimetry, fluorescence or luminescence,
- chromophores,
- groups with an electron density detectable by electron microscopy or by virtue of their electrical property,
- electrochemically detectable groups,
- derivatives of an iron complex,
- radioactive molecules,
- ligand/anti-ligand pairs selected among the following pairs: biotin/streptavidin, hapten/antibody, antigen/antibody, peptide/antibody, sugar/lectin, polynucleotide/sequence complementary to the polynucleotide,
- fluorescent compounds with a molecular weight of less than 1000 g/mol,
- haptens with a molecular weight of less than 1000 g/mol,
- fluorophores,
• R₂ and R₃ independently of each other represent: H, CONHR, COOR with R corresponding to a saturated aliphatic hydrocarbon group containing from one to six carbon atoms or a monocyclic aromatic group, and
• L is a linking arm comprising a linear chain of at least two covalent bonds comprising an -(O-CH₂-CH₂)- moiety, repeated from 1 to 20 times.

2. Labelling reagent of formula (D): 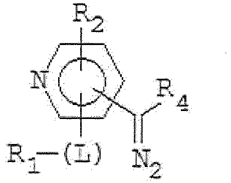 in which:
• R₁ represents a detectable label capable of directly or indirectly generating a detectable signal and selected among:
- enzymes which produce a detectable signal by colorimetry, fluorescence or luminescence,
- chromophores,
- groups with an electron density detectable by electron microscopy or by virtue of their electrical property,
- electrochemically detectable groups,
- derivatives of an iron complex,
- radioactive molecules,
- ligand/anti-ligand pairs selected among the following pairs: biotin/streptavidin, hapten/antibody, antigen/antibody, peptide/antibody, sugar/lectin, polynucleotide/sequence complementary to the polynucleotide,
- fluorescent compounds with a molecular weight of less than 1000 g/mol,
- haptens with a molecular weight of less than 1000 g/mol,
- fluorophores,
• R₂ represents: H, CONHR, COOR with R corresponding to a saturated aliphatic hydrocarbon group containing from one to six carbon atoms or a monocyclic aromatic group,
• R₄ represents: H, a saturated aliphatic hydrocarbon group containing from one to six carbon atoms or a monocyclic aromatic group, and
• L is a linking arm comprising a linear chain of at least two covalent bonds comprising an -(O-CH₂-CH₂)- moiety, repeated from 1 to 20 times.

3. Labelling reagent of formula (E): 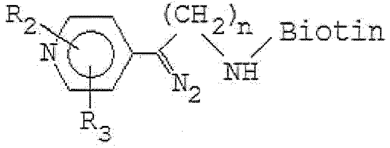 in which:
• R₂ and R₃ independently of each other represent: H, CONHR, COOR with R corresponding to a saturated aliphatic hydrocarbon group containing from one to six carbon atoms or a monocyclic aromatic group, and
• n is a whole number lying between 1 and 12.

4. Labelling reagent of formula (F): 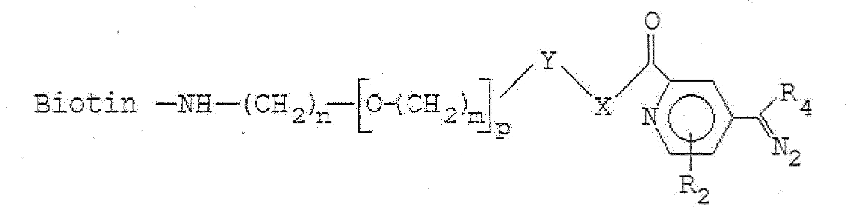 in which:
• R₂ represents: H, CONHR, COOR with R corresponding to a saturated aliphatic hydrocarbon group containing from one to six carbon atoms or a monocyclic aromatic group,
• R₄ represents: H, a saturated aliphatic hydrocarbon group containing from one to six carbon atoms or a monocyclic aromatic group,
• -Y-X represents -CH₂NH-, -CONH-, -NHCO-, -CH₂O- or -CH₂S-, and
• m, n and p are each, independently of each other, a whole number lying between 1 and 12.

5. Labelling reagent according to Claim 3, of formula (G) :

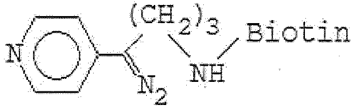

6. Labelling reagent according to Claim 4, of formula (H):

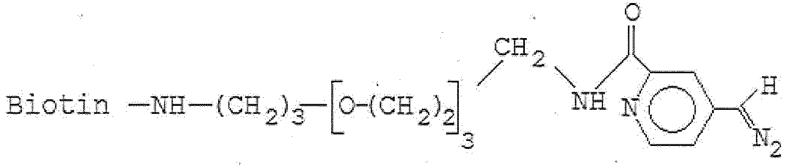

7. Reagent according to either one of Claims 1 to 2, **characterized in that** R₁ is made up of a D-biotin residue of formula (I):

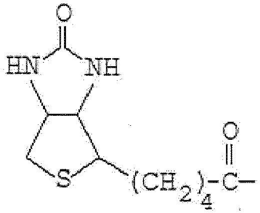

8. Process for synthesis of a labelling reagent according to any one of Claims 1, 3 and 5, comprising the following stages:
a) an aromatic carboxylic acid derivative is reacted with the enolate of a lactone (Claisen type reaction) to form a cyclic precursor,
b) which cyclic precursor is then opened with a halo acid to form an aromatic halo ketone,
c) the carbonyl function of the aromatic halo ketone is protected by a protective group to form a protected precursor,
d) which protected precursor is subjected to an amination reaction (of the Gabriel type) to form an aminated precursor,
e) which aminated precursor is deprotected to liberate the amine function, the said amine function being reacted with a detectable label the carboxyl function whereof is activated to form a precursor containing a detectable label,
f) the labelled precursor is subjected to a reaction of deprotection of the carbonyl function to form a labelled and carbonyl-containing precursor, and finally
g) the labelled and carbonyl-containing precursor is transformed into a labelling reagent, such as described above, by transformation of the carbonyl function into a diazo function.

9. Process for synthesis of a labelling reagent according to any one of Claims 2, 4 and 6, comprising the following stages:
a) an aromatic dicarboxylic acid is esterified to form a diester,
b) this diester is regioselectively substituted with a compound derived from mono-protected aminated ethylene glycol to form a precursor protected on the carboxyl function and the amine function,
c) which precursor is then reduced to the alcohol and oxidised to the aldehyde,
d) the aldehyde synthesised in stage c) is subjected to an acid treatment in order concomitantly to protect this function while deprotecting the amine function, to lead to an aminated acetal,
e) which aminated acetal is reacted with a detectable label the carboxyl function whereof is activated to form a precursor containing a detectable label,
f) the labelled precursor is subjected to a reaction of deprotection of the carbonyl function to form a labelled and carbonyl-containing precursor, and finally
g) the labelled and carbonyl-containing precursor is transformed into a labelling reagent, such as described above, by transformation of the carbonyl function into a diazo function by a Bamford Stevens type reaction.

10. Process for the labelling of a biological molecule, comprising the contacting of a biological molecule and a reagent according to any one of Claims 1 to 7 in homogeneous solution, in an aqueous buffer containing at least 50% of water.

11. Process for labelling and fragmentation of a single or double strand nucleic acid comprising the following stages:
• fragmenting the nucleic acid,
• attaching a label onto at least one of the fragments by means of a labelling reagent selected from the reagents according to any one of Claims 1 to 7,
the said reagent coupling covalently and mainly onto at least one phosphate of the said fragment.

12. Process according to Claim 11, **characterized in that** the fragmentation and the labelling are effected in two stages.

13. Process according to Claim 11, **characterized in that** the fragmentation and the labelling are effected in one stage.

14. Process according to any one of Claims 11 to 13, **characterized in that** the labelling is effected in aqueous homogeneous solution containing at least 50% of water.

15. Process according to any one of Claims 11 to 13, **characterized in that** the fragmentation is effected by an enzymatic, physical or chemical route.

16. Kit for detection of a target nucleic acid containing a labelled nucleic acid obtained by the process according to any one of Claims 11 to 15.

17. Solid support onto which is attached a reagent according to any one of Claims 1 to 7.

18. Process for capture of nucleic acids comprising the following stages:
• a solid support is used onto which there is directly or indirectly attached at least one biological molecule obtained according to the process according to Claim 10, or a nucleic acid obtained by the process according to any one of Claims 11 to 15, the biological molecule or the nucleic acid containing a diazomethyl function,
• it is contacted with a biological sample capable of containing free nucleic acids, and
• the solid support where the molecule(s) are covalently attached to at least one nucleic acid is washed.
